# EUROPÄISCHE PATENTSCHRIFT

(11) **EP 2 072 584 B1**
(45) Veröffentlichungstag und Bekanntmachung des Hinweises auf die Patenterteilung: **11.05.2011**
(21) Anmeldenummer: 07150050.8
(22) Anmeldetag: 17.12.2007
(51) Int. Cl.: C09B 47/067, C09B 47/30, A61K 31/40, A61K 31/555, A61K 41/00

(54) **Phthalocyanin-Farbstoffe, ihre Herstellung und Verwendung**
Phthalocyanine dyes, their preparation and use
Colorants phthalocyanines, leur préparation et leur utilisation

(43) Veröffentlichungstag der Anmeldung: 24.06.2009
(73) Patentinhaber: ILFORD Imaging Switzerland GmbH, 1723 Marly 1 (CH)
(72) Erfinder: Baettig, Kurt, 1724, Le Mouret (CH); Moigno, Damien, 1723, Marly (CH)

(56) Entgegenhaltungen:
- EP-A- 1 867 685
- WO-A-95/05818
- JP-A- 9 249 814
- JP-A- 2003 213 153

## Beschreibung

### Technisches Gebiet

Die Erfindung bezieht sich auf neue substituierte, wasserlösliche Phthalocyanin-Farbstoffe, ihre Salze, ein Verfahren zu ihrer Herstellung und ihre Verwendung in pharmazeutischen Zusammensetzungen und Arzneimitteln für die photodynamische Therapie und zur Behandlung von durch Viren, Pilzen oder Bakterien ausgelösten Infektionskrankheiten, Krebs und dermatologischen Erkrankungen.

### Stand der Technik

Unter der photodynamischen Therapie versteht man ein Verfahren zur Behandlung von Tumoren und anderen Gewebeveränderungen mit Licht in Kombination mit einer lichtempfindlichen Substanz, einem so genannten Photosensibilisator, und im Gewebe vorhandenem Sauerstoff. Dazu wird dem Patienten ein solcher Sensibilisator oder einer seiner Stoffwechselvorläufer verabreicht, der sich selektiv im Tumor anreichert. Nach einer gewissen Wartezeit wird anschließend der Tumor und das ihn umgebende gesunde Gewebe mit Licht geeigneter Wellenlänge bestrahlt. Dabei werden durch photophysikalische Prozesse aus dem im Gewebe vorhandenen Sauerstoff toxische Substanzen, sogenannte reaktive Sauerstoff-Spezies erzeugt, die aufgrund der Tumorselektivität des Sensibilisators gezielt den Tumor schädigen.

Gegenüber einer chirurgischen Behandlung bietet die photodynamische Therapie den Vorteil eines nicht beziehungsweise minimal-invasiven Verfahrens. Insbesondere entfällt die aus Sicherheitsgründen erforderliche weiträumige Entfernung von gesundem Gewebe in der Tumorumgebung. Eine Bestrahlung dauert etwa 10 Minuten bis 100 Minuten bei typischen Bestrahlungsstärken von 100 mW / cm² bis 200 mW / cm². Die Erwärmung des Gewebes ist daher nur gering. Die photodynamische Therapie von Tumoren erfolgt im Normalfall in einer einmaligen Bestrahlungssitzung, jedoch besteht durchaus die Möglichkeit der Wiederholung. Da die Bestrahlung mit normalem Licht geschieht, ist die Belastung der Patienten im Vergleich zu den "klassischen" Therapieverfahren relativ gering.

Der Nachteil der photodynamischen Therapie ist im wesentlichen die geringe Eindringtiefe von Licht von nur einigen Millimetern, so dass in der Regel nur nicht zu fortgeschrittene oder flächig wachsende Tumore erfolgreich therapiert werden können. Daher bieten sich vor allem beispielsweise Hauttumore, wie aktinische Keratosen, das Basaliom, aber auch Warzen als Einsatzgebiet für die photodynamische Therapie an. Durch den Einsatz von Lasern in Kombination mit Lichtleitfasern lassen sich auch Tumore an endoskopisch zugänglichen körperinneren Oberflächen behandeln.

Die photodynamische Therapie von inneren Tumoren ist bislang wenig verbreitet und wird meist nur palliativ eingesetzt wie beispielsweise in der Speiseröhre, bei Gallengangs- und Gallenblasenkarzinom oder bei Gehirntumoren.

Obwohl die photodynamische Therapie bereits zu Beginn des 20. Jahrhunderts in München untersucht wurde, erlangte es erst in den 80er Jahren durch eine Verbesserung der Photosensibilisatoren und den Einsatz von Lasern eine gewisse Verbreitung. Typische Einsatzgebiete sind Tumoren in der Harnblase, im äußeren Kopfbereich, in Mundhöhle, Kehlkopf, Speiseröhre, in der Lunge, im Gallengang sowie im Genitalbereich.

Der eigentliche photophysikalische Prozess der photodynamischen Therapie verläuft in mehreren Schritten und erfordert die Anwesenheit von Sauerstoff, der in den meisten Zellen in ausreichender Menge vorhanden ist. Ein Molekül des Photosensibilisators absorbiert ein Photon des Lichtes und wird in den ersten angeregten Singlett-Zustand angehoben. Je grösser die Lebensdauer dieses Singlett-Zustands ist, umso grösser ist auch die Wahrscheinlichkeit für den eher seltenen Übergang durch Interkombination in einen ebenfalls angeregten Triplett-Zustand. Da optische Übergänge dieses Triplett-Zustands in den Grundzustand sehr unwahrscheinlich sind, hat er eine ungewöhnlich grosse Lebensdauer. Das ermöglicht den Kontakt mit besonders vielen Molekülen der Umgebung. Trifft er dabei ein Molekül, dessen Grundzustand ein Triplett-Zustand ist, so ist ein Energieaustausch möglich, wobei beide Moleküle in den Singlett-Zustand übergehen. Eines der wenigen Moleküle mit einem Triplett-Grundzustand ist molekularer Sauerstoff. Da die Energie des angeregten Sensibilisatormoleküls grösser ist als die für einen Übergang des Sauerstoffs in einen angeregten Singlett-Zustand erforderliche, kann dieser Energieaustausch stattfinden. Der dabei entstehende Singlett-Sauerstoff hat wiederum eine besonders grosse Lebensdauer hinsichtlich eines optischen Übergangs in den Grundzustand. Aufgrund seiner chemischen Reaktionsfreudigkeit kann er jedoch Zellbestandteile in der Umgebung durch Oxidation schädigen. Dadurch kann er eine Nekrose oder - durch Wirkung auf die Mitochondrienmembran - eine Apoptose auslösen.

Als Photosensibilisatoren werden überwiegend Porphyrine eingesetzt, die sich bei Bestrahlung mit rotem Licht mit einer Wellenlänge zwischen 630 nm und 635 nm aktivieren lassen. Oft wird auch 5-Aminolävulinsäure oder deren Methylester eingesetzt, Stoffwechselvorläufer des Protoporphyrins, der selektiv in Tumorzellen eine Porphyrinsynthese anregt. Neuere Sensibilisatoren lassen sich bei noch höheren Wellenlängen anregen mit dem Vorteil einer grösseren Eindringtiefe des Lichtes in das Gewebe. Photosensibilisatoren fluoreszieren in der Regel und werden daher auch in der Fluoreszenzdiagnose von Tumoren eingesetzt.

Die optimalen Wellenlängen für die photodynamische Therapie liegen zwischen 600 nm und 1500 nm. Bei kürzeren Wellenlängen ist die Durchlässigkeit des Gewebes für die Strahlung zu gering und bei höheren Wellenlängen ist die Energie der Strahlung zu gering. Bei einer Wellenlänge von 630 nm dringt das Licht 4 mm tief ins Gewebe ein, bei einer Wellenlänge von 700 nm wären es 8 mm.

Die Verwendung von Phthalocyanin-Farbstoffen als Photosensibilisatoren in der photodynamischen Therapie wurde zuerst von E. Ben-Hur und I. Rosenthal in "The phthalocyanines: a new class of mammalian cells photosensitizers with a potential for cancer phototherapy", International Journal of Radiation Biology 47, 145-147 (1985) und nachher von C. M. Allen, W. M. Sharman und J. E. van Lier in "Current status of phthalocyanines in the photodynamic therapy of cancer", Journal of Porphyrins and Phthalocyanines 5, 161 - 169 (2002) sowie von E. Ben-Hur und W.-S. Chan in "Phthalocyanines in Photobiology and their Medical Applications", Band 19, Seiten 1 - 35 von K. M. Kadish, K. M. Smith und R. Guilard (Herausgeber), "The Porphyrin Handbook", Academic Press San Diego (2003), ISBN 0-12-393229-7 beschrieben. Die photodynamische Aktivität und die Adsorption von sulfonierten Phthalocyaninen an Membranen wird von A. A. Pashkovskaya, E. A. Sokolenko, V. S. Sokolov, E. A. Kotova und Y. N. Antonenko in "Photodynamic activity and binding of sulfonated metallophthalocyanines to phospholipid membranes: Contribution of metal-phosphate coordination", Biochimica et Biophysica Acta 1768, 2459 - 2465 (2007) beschrieben.

In der Patentanmeldung EP 1'867'685 (Stand der Technik gemäss Artikel 54(3) EPÜ) werden Phthalocyanin-Farbstoffe der allgemeinen Formeln (II) und (III), worin
Me für Kupfer, Zink, Nickel, Mangan, Kobalt, Aluminium oder Vanadium steht,
als Farbstoffe mit verbesserter Ozonbeständigkeit für den Tintenstrahldruck beschrieben.

Die Herstellung und photodynamische Aktivität des oft verwendeten Zink-Phthalocyanin-Farbstoffs der Formel (I) als Photosensibilisator 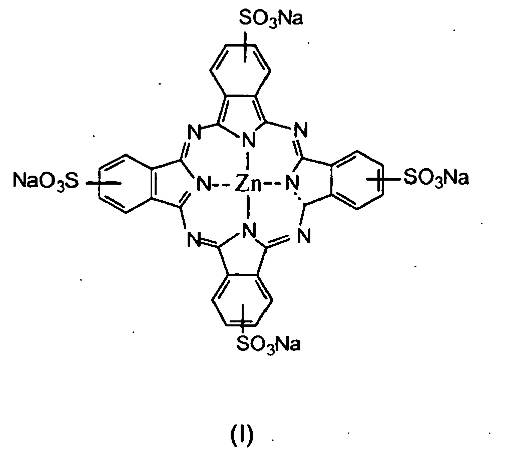 wird von J. Griffith, J. Schoefield, M. Wainwright und S. B. Brown in "Some observations on the Synthesis of Polysubstituted Zinc Phthalocyanine Sensitisers for Photodynamic Therapies", Dyes and Pigments 33, 65 - 78 (1997) beschrieben.

In der Patentanmeldung WO 92/01'753 werden unsubstituierte Phthalocyanin-Farbstoffe als Photosensibilisatoren für die photodynamische Therapie beschrieben. Das Zentralatom des Phthalocyanin-Farbstoffs ist Silizium oder Aluminium.

In der Patentanmeldung WO 95/06'688 werden ebenfalls unsubstituierte Phthalocyanin-Farbstoffe für die photodynamische Therapie beschrieben. Das Zentralatom des Phthalocyanin-Farbstoffs ist Silizium oder Aluminium.

Im Patent US 5'484'778 werden ebenfalls unsubstituierte Phthalocyanin-Farbstoffe als Photosensibilisatoren für die photodynamische Therapie beschrieben. Das Zentralatom des Phthalocyanin-Farbstoffs ist Silizium.

In der Patentanmeldung WO 95/05'818 werden substituierte Phthalocyanin-Farbstoffe als Photosensibilisatoren für die photodynamische Therapie beschrieben. Das Zentralatom des Phthalocyanin-Farbstoffs ist entweder ein diamagnetisches Metallatom oder Silizium.

In der Patentanmeldung WO 02/090'361 werden substituierte Phthalocyanin-Farbstoffe als Photosensibilisatoren für die photodynamische Therapie beschrieben. Das Zentralatom des Phthalocyanin-Farbstoffs ist Silizium, Zink oder Aluminium.

Ein idealer Photosensibilisator für die photodynamische Therapie sollte im Zellmilieu gut löslich, nicht toxisch und hoch wirksam sein und sich selektiv möglichst nur in den Tumoren anreichern. Er sollte sich auch, falls er toxisch ist, rasch zu harmlosen, nicht toxischen Verbindungen abbauen, welche vom Körper leicht ausgeschieden werden können.

Es besteht deshalb weiterhin ein Bedarf für verbesserte Photosensibilisatoren für die photodynamische Therapie, welche die obigen Anforderungen erfüllen. Insbesondere sollte das Absorptionsmaximum der Substanzen oberhalb von 630 nm, vorzugsweise oberhalb von 650 nm liegen.

### Beschreibung der Erfindung

Wir haben nun überraschend gefunden, dass neue, substituierte Phthalocyanin-Farbstoffe diese Bedingungen erfüllen und damit ideale Photosensibilisatoren für die photodynamische Therapie sind.

Ein weiteres Ziel der Erfindung ist die Bereitstellung von pharmazeutischen Zusammensetzungen und Arzneimitteln für die photodynamische Therapie, welche die erfindungsgemässen Phthalocyanin-Farbstoffe enthalten.

Die vorliegende Erfindung bezieht sich auf neue Phthalocyanin-Farbstoffe der allgemeinen Formel (II) 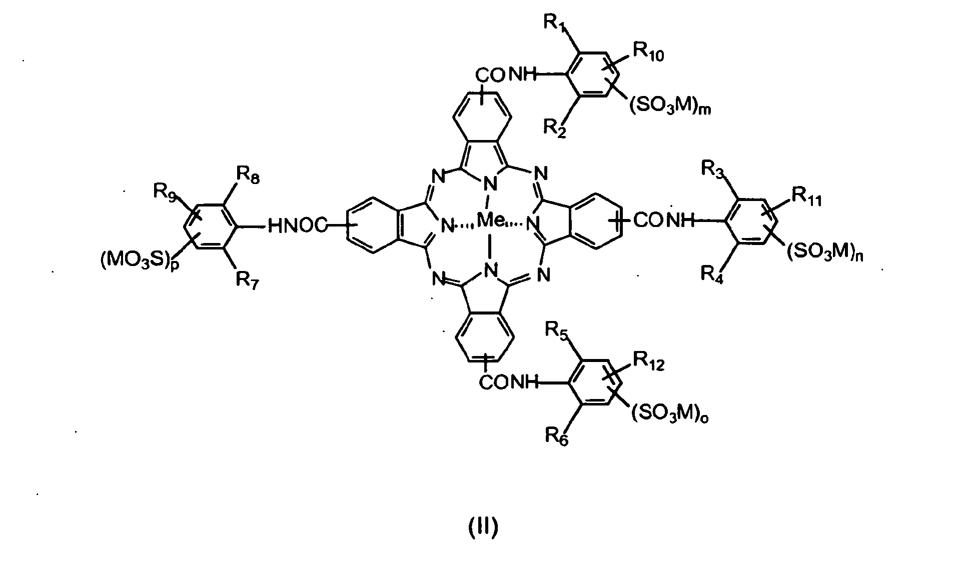 worin
- R₁ - R₈: unabhängig voneinander für einen linearen oder verzweigten Alkylrest mit bis zu 8 Kohlenstoffatomen;
- R₉-R₁₂: unabhängig voneinander für Wasserstoff, einen linearen oder einen verzweigten Alkylrest oder Alkoxylrest mit bis zu 8 Kohlenstoffatomen oder ein Halogenatom;
- Me: für Magnesium, Wasserstoff, Gallium, Germanium oder Zinn;
- M: für Wasserstoff, ein Metallkation oder ein Ammoniumkation, das gege- benenfalls einen oder mehrere unsubstituierte oder substituierte Alkyl- reste oder Hydroxyalkoxyalkylreste mit jeweils 1 bis 12 Kohlenstoffato- men aufweist, stehen
- und:
- m, n , o, p: unabhängig voneinander den Wert 1 oder 2 annehmen.

Bevorzugt sind die Phthalocyanin-Farbstoffe, bei denen m, n, o und p den Wert 1 annehmen.

Bevorzugt sind auch die Phthalocyanin-Farbstoffe, bei denen Me für Magnesium oder Wasserstoff steht.

Ebenfalls bevorzugt sind die Phthalocyanin-Farbstoffe, bei denen M das Kation der Metalle Natrium, Kalium oder Lithium oder ein Ammoniumkation ist, das einen oder mehrere unsubstituierte oder substituierte Alkylreste oder Hydroxyalkoxyalkylreste mit jeweils 1 bis 6 Kohlenstoffatomen aufweist.

Besonders bevorzugt sind die symmetrischen Phthalocyanin-Farbstoffe der allgemeinen Formel (III), die um das Zentralatom Me herum 4 gleiche Gruppen angeordnet haben. In diesen Farbstoffen nehmen m, n, o und p den gleichen Wert an. Die Substituenten R₁, R₃, R₅ und R₇ sind identisch, ebenso die Substituenten R₂, R₄, R₆ und R₈ wie auch die Substituenten R₉**,** R₁₀, R₁₁ und R₁₂. R₁, R₂, R₁₀, Me und m haben die gleiche Bedeutung wie vorhin angegeben. 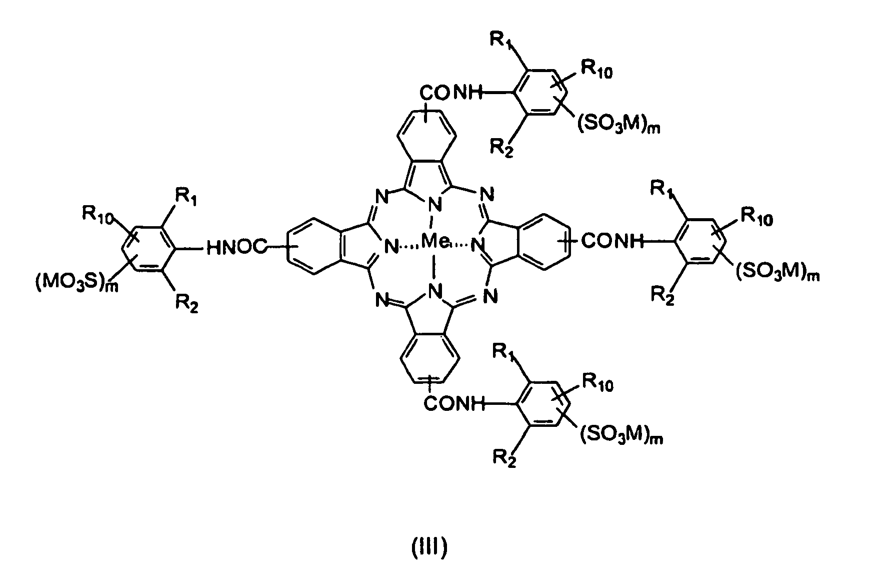 Bevorzugt sind auch hier die symmetrischen Phthalocyanin-Farbstoffe, bei denen m den Wert 1 annimmt.

Besonders bevorzugt sind ebenfalls die symmetrischen Phthalocyanin-Farbstoffe, bei denen Me für Magnesium oder Wasserstoff steht.

Ebenfalls bevorzugt sind die symmetrischen Phthalocyanin-Farbstoffe, bei denen M das Kation der Metalle Natrium, Kalium oder Lithium oder ein Ammoniumkation ist, das einen oder mehrere unsubstituierte oder substituierte Alkylreste oder Hydroxyalkoxyalkylreste mit jeweils 1 bis 6 Kohlenstoffatomen aufweist.

Die Erfindung bezieht sich auch auf ein Verfahren zur Herstellung der erfindungsgemässen Phthalocyanin-Farbstoffe der allgemeinen Formel (II), das dadurch gekennzeichnet ist, dass
ein aromatisches Amin der allgemeinen Formel (IV A), 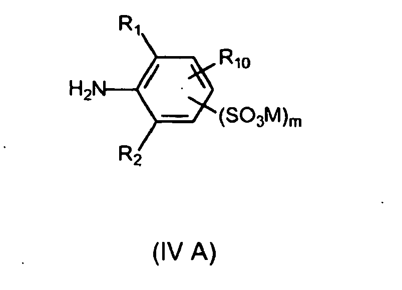 worin R₁, R₂, R₁₀ und m die gleiche Bedeutung wie vorhin angegeben haben,
der allgemeinen Formel (IV B), 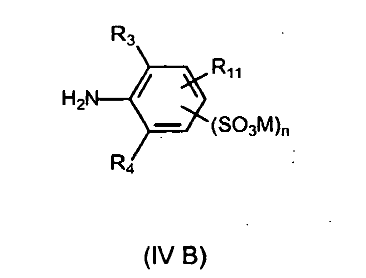 worin R₃, R₄, R₁₁ und n die gleiche Bedeutung wie vorhin angegeben haben,
der allgemeinen Formel (IV C), 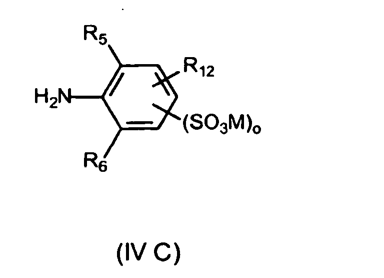 worin R₅, R₆, R₁₂ und o die gleiche Bedeutung wie vorhin angegeben haben,
und
der allgemeinen Formel (IV D), 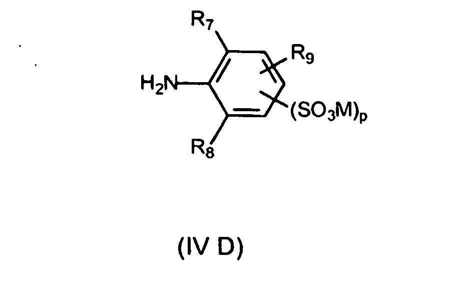 worin R₇, R₈, R₁₂ und p die gleiche Bedeutung wie vorhin haben,
mit dem Carbonsäurechlorid der Formel (V) 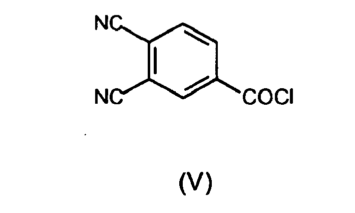 unter Bedingungen umgesetzt werden, dass die Amide der allgemeinen Formeln 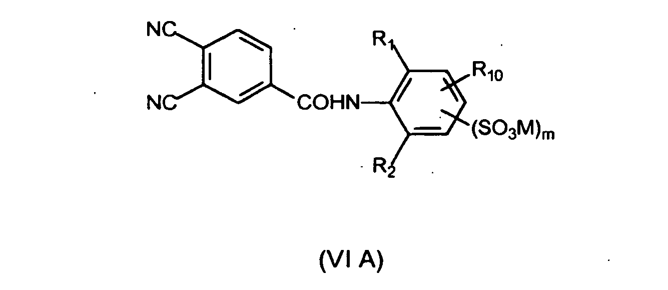 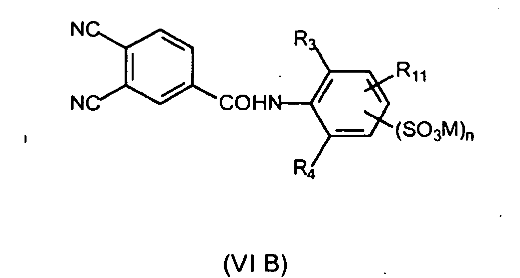 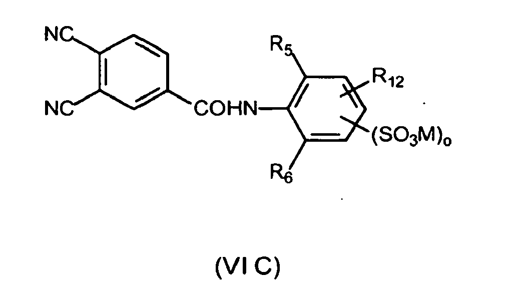 und 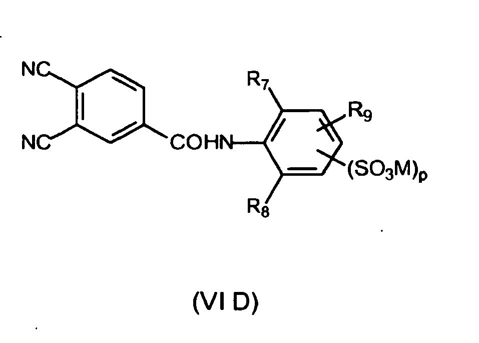 gebildet werden.

Zur Herstellung der asymmetrischen Phthalocyanin-Farbstoffe der allgemeinen Formel (II) wird ein Gemisch der Amide (VI A), (VI B), (VI C) und (VI D) mit einem Salz des Metalls Me, falls Me nicht für Wasserstoff steht, bei höheren Temperaturen in einem aprotischen Lösungsmittel in Gegenwart einer starken Base umgesetzt.

Besonders bevorzugte aprotische Lösungsmittel sind N-Methylpyrrolidon, N-Methylacetamid und Formamid. Andere aprotische Lösungsmittel mit ähnlichen Eigenschaften können ebenfalls verwendet werden.

Geeignete Metallsalze sind die Acetate, Nitrate und Chloride.

Geeignete starke Basen sind 1,8-Diazabicyclo(4.4.0)undec-7-en, 1,4-Diazabicyclo(2.2.2)octan und weitere Verbindungen mit ähnlichen basischen Eigenschaften.

Geeignete Bedingungen für die Umsetzung werden beispielsweise von M. Hanack, H. Heckmann und R. Polley in "Phthalocyanines and Related Compounds", Band E 9d, Seiten 717 - 842 von Houben-Weyl, "Methods of Organic Chemistry", Georg Thieme Verlag Stuttgart · New York (1998), ISBN 3-13-101614-0 beschrieben.

Zur Herstellung der symmetrischen Phthalocyanin-Farbstoffe der allgemeinen Formel (III) wird das Amid der allgemeinen Formel (VI A) unter den oben beschriebenen Bedingungen mit einem Salz des Metalls Me umgesetzt, falls Me nicht für Wasserstoff steht.

Spezifische Beispiele von symmetrischen Phthalocyanin-Farbstoffen der allgemeinen Formel (III) sind die folgenden, wobei die Substituenten Me und M in Tabelle 1 spezifiziert werden: 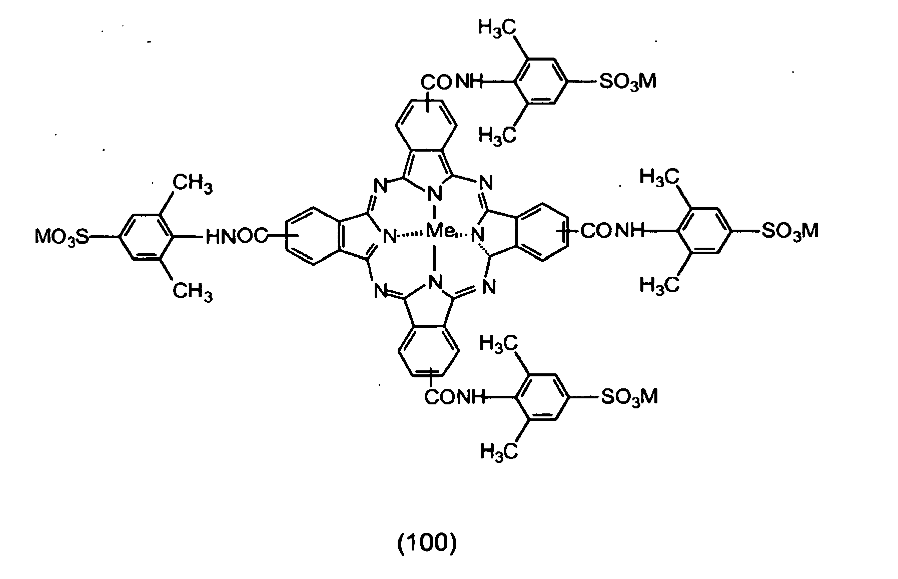 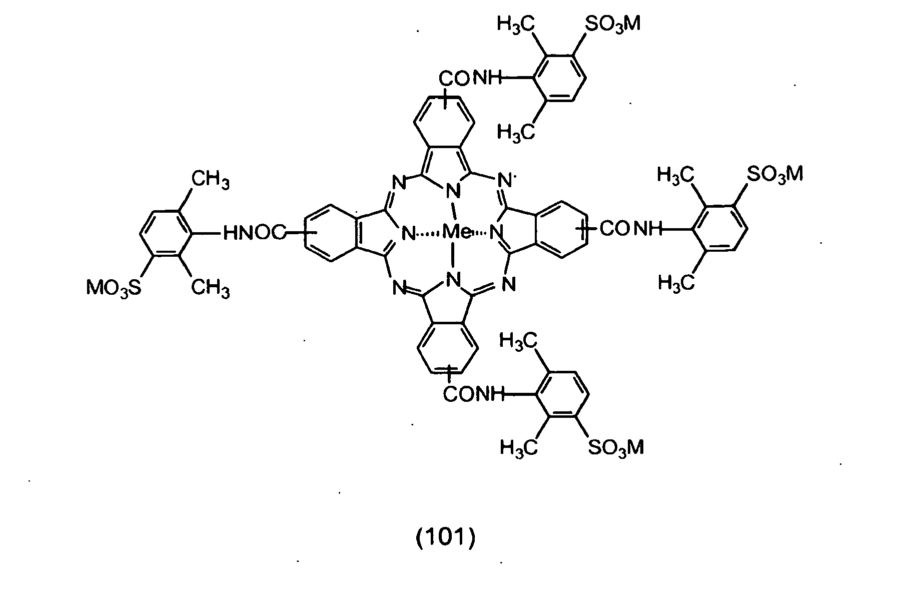 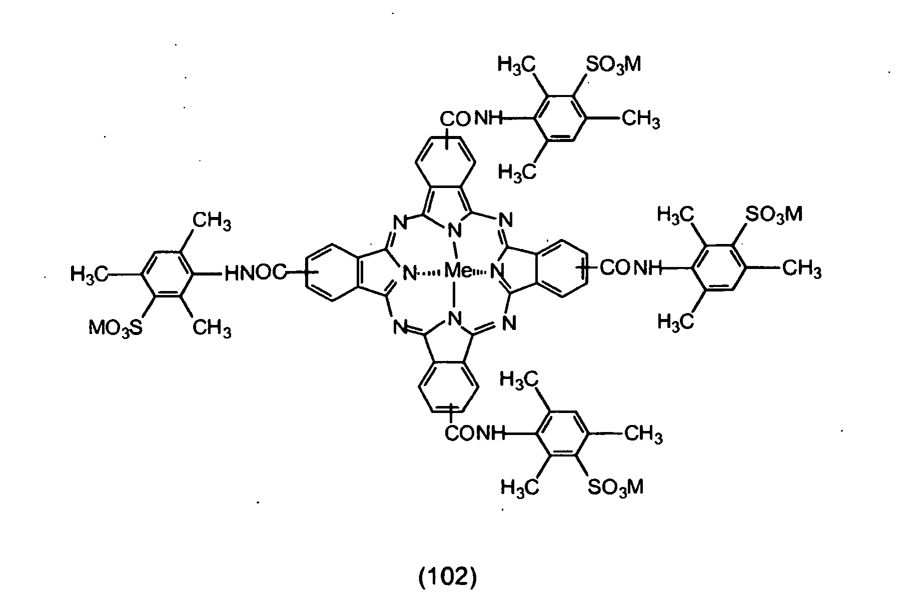 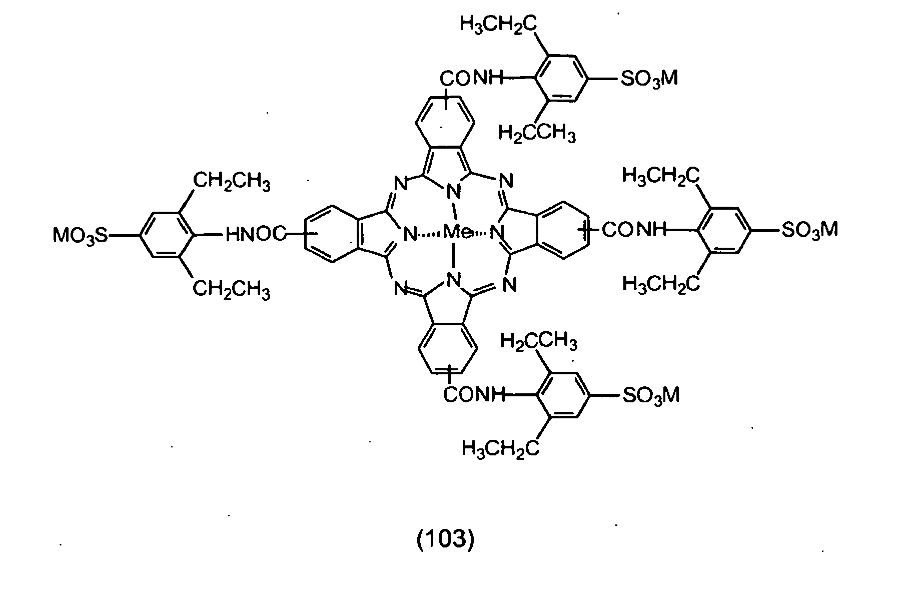 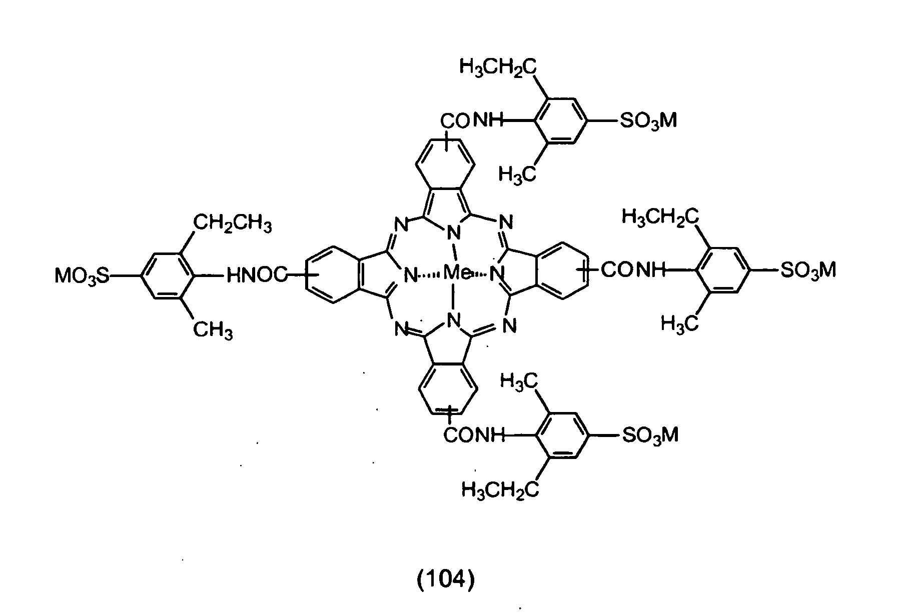 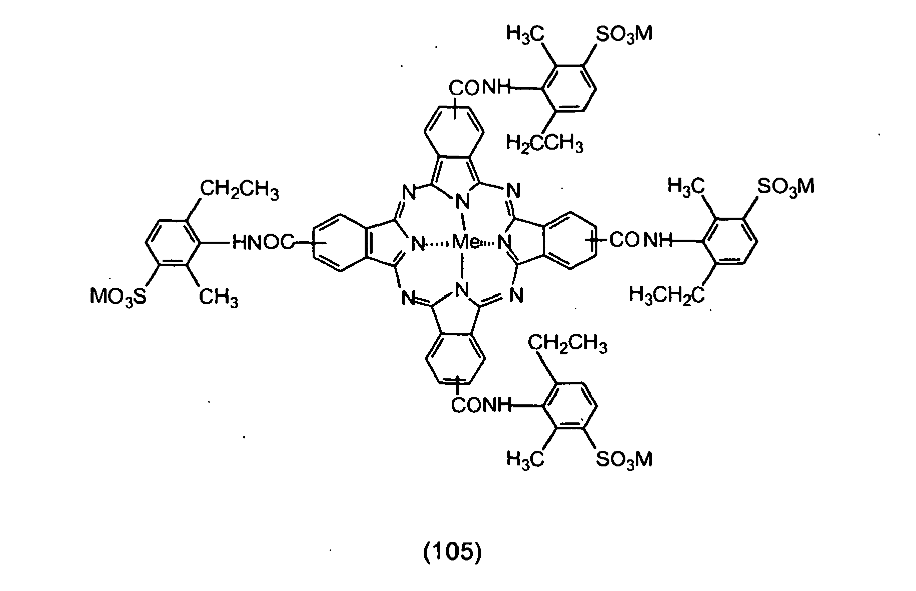 und

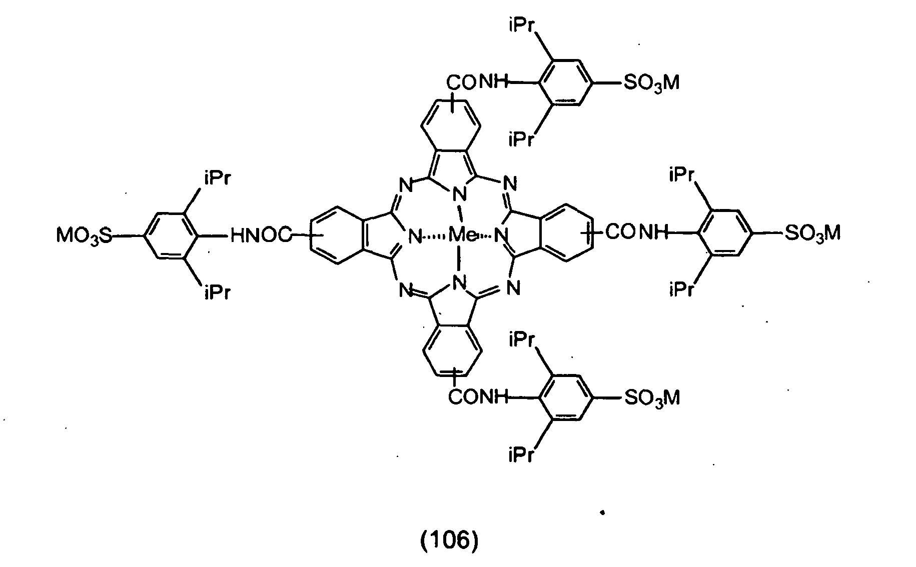

Weiter betrifft die Erfindung nicht nur reine Phthalocyanin-Farbstoffe der allgemeinen Formeln (II) und (III), sondern auch Gemische dieser Phthalocyanin-Farbstoffe.

Die erfindungsgemässen Phthalocyanin-Farbstoffe der allgemeinen Formeln (II) und (III) sowie die Phthalocyanin-Farbstoffe der allgemeinen Formeln (II) und (III), bei denen Me für Aluminium oder Zink steht, sind sehr geeignet als Photosensibilisatoren für die photodynamische Therapie der verschiedenartigsten Tumore sowie ebenfalls für die Therapie von bakteriellen und viralen Infektionen. Sie sind auch besonders geeignet als photodiagnostische Reagenzien.

Die erfindungsgemässen Phthalocyanin-Farbstoffe sowie die Phthalocyanin-Farbstoffe der allgemeinen Formeln (II) und (III), bei denen Me für Aluminium oder Zink steht, können zur Zerstörung von Krebsgewebe, von Krebsvorstufen, lichtgeschädigten oder anderweitig geschädigten sowie pathogenen Zellen, von angeregten Zellen wie Lymphozyten oder andere Zellen des Immunsystems, und entzündeten Zellen verwendet werden wie auch zur Behandlung von durch Viren, Pilzen oder Bakterien ausgelösten Infektionskrankheiten, Krebs und dermatologischen Erkrankungen.

Die erfindungsgemässen Phthalocyanin-Farbstoffe sowie die Phthalocyanin-Farbstoffe der allgemeinen Formeln (II) und (III), bei denen Me für Aluminium oder Zink steht, können durch Licht mit Wellenlängen oberhalb von etwa 670 nm aktiviert werden, welches relativ gut in das Körpergewebe eindringen kann.

Die hydrophilen Eigenschaften der Substituenten und/oder die Absorption auf hydrophilen Trägem können den Abbau der erfindungsgemässen Phthalocyanin-Farbstoffe sowie die Phthalocyanin-Farbstoffe der allgemeinen Formeln (II) und (III), bei denen Me für Aluminium oder Zink steht, im Körper fördern. Dadurch werden die erfindungsgemässen Phthalocyanin-Farbstoffe sowie die Phthalocyanin-Farbstoffe der allgemeinen Formeln (II) und (III), bei denen Me für Aluminium oder Zink steht, in vivo rasch aus dem Körper ausgeschieden und die Probleme der Toxizität sowie Hautphototoxizität werden drastisch reduziert.

Die erfindungsgemässen Phthalocyanin-Farbstoffe der allgemeinen Formeln (II) und (III) sowie die Phthalocyanin-Farbstoffe der allgemeinen Formeln (II) und (III), bei denen Me für Aluminium oder Zink steht, werden vorzugsweise in pharmazeutischen Zusammensetzungen und Arzneimitteln eingesetzt, welche eine pharmazeutische Trägersubstanz wie eine Mischung von Polyethylenglykol, Ethanol und Wasser; Polyethylenglykol und Wasser, Liposome, Nanocontainer, Biopolymere oder andere geeignete pharmazeutisch zulässige Trägersubstanzen und geeignete Verdünnungsmittel enthalten.

Diese pharmazeutischen Zusammensetzungen und Arzneimittel, welche die erfindungsgemässen Phthalocyanin-Farbstoffe der allgemeinen Formeln (II) und (III) sowie die Phthalocyanin-Farbstoffe der allgemeinen Formeln (II) und (III), bei denen Me für Aluminium oder Zink steht, enthalten, sind sehr geeignet als photosensibilisatorhaltige Zusammensetzungen und Arzneimittel für die photodynamische Therapie der verschiedenartigsten Tumore sowie ebenfalls für die Therapie von bakteriellen und viralen Infektionen. Sie sind auch besonders geeignet als photodiagnostische Reagenzien.

Diese pharmazeutischen Zusammensetzungen und Arzneimittel, welche die erfindungsgemässen Phthalocyanin-Farbstoffe der allgemeinen Formeln (II) und (III) sowie die Phthalocyanin-Farbstoffe der allgemeinen Formeln (II) und (III), bei denen Me für Aluminium oder Zink steht, enthalten, können zur Zerstörung von Krebsgewebe, von Krebsvorstufen, lichtgeschädigten oder anderweitig geschädigten sowie pathogenen Zellen, von angeregten Zellen wie Lymphozyten oder andere Zellen des Immunsystems, und entzündeten Zellen verwendet werden wie auch zur Behandlung von durch Viren, Pilzen oder Bakterien ausgelösten Infektionskrankheiten, Krebs und dermatologischen Erkrankungen.

Die vorliegende Erfindung wird durch die folgenden Beispiele näher beschrieben, ohne dass dadurch die beanspruchten Phthalocyanin-Farbstoffe in irgendeiner Weise eingeschränkt werden.

### Beispiele

### Beispiel 1 (Farbstoff des Stands der Technik)

Der symmetrische Zink-Phthalocyanin-Farbstoff (100) wurde folgendermassen hergestellt:
6.7 g (30 mMol) des Anilins der Formel (VII), hergestellt nach dem von G. P. Britovsek, G. Y. Y. Woo und N. Assavathorn in "Synthesis and reactivity of water-soluble platinum(II) complexes containing nitrogen ligands", Journal of Organometallic Chemistry 679, 110 - 115 (2003) beschriebenen Verfahren, 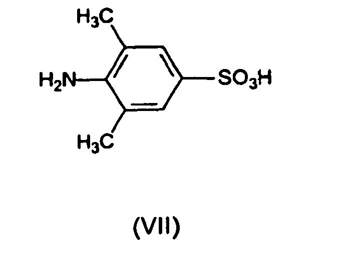 werden in 25 ml Pyridin suspendiert.

Zu dieser Suspension wird unter Rühren während 10 Minuten 5.7 g (30 mmol) des Säurechlorids der Formel (VIII), hergestellt nach dem von J. J. He, G. Benkö, F. Korodi, T. Polivka, R. Lomoth, B. Ȧkermak. L. Sun, A Hagfeldt und V. Sundström in "Modified Phthalocyanines for Efficient Near-IR Sensitization of Nanostructured TiO2 Electrode", Journal of the American Chemical Society 124, 4922 - 4932 (2002) beschriebenen Verfahren, 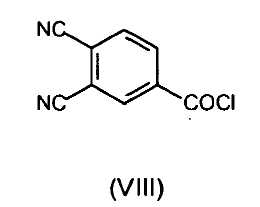 gelöst in 8 ml Ethylacetat, unter Kühlung mit Eis so zugetropft, dass die Temperatur 30° C nicht übersteigt. Nach Ende der Zugabe wird der Ansatz 18 Stunden bei Raumtemperatur nachgerührt. Anschliessend wird das Gemisch mit 40 ml Ethyl acetat verdünnt und das ausgefallene Produkt abgenutscht. Das Rohprodukt wird durch Umkristallisation in 140 ml Methanol gereinigt. Nach Trocknung erhält man 7.2 g des Amids der Formel (IX) 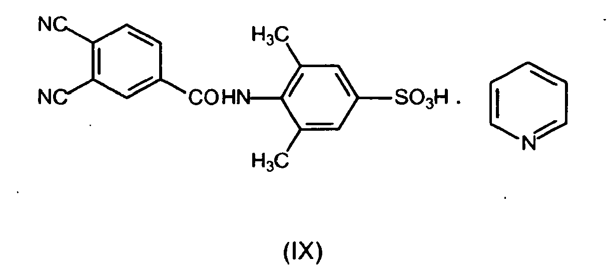 7.0 g (16 mMol) des Amids der Formel (IX), 0.88 g (4 mmol) Zinkacetat-Dihydrat und 4.0 g 1,8-Diazabicyclo(4.4.0)undec-7-en in 10 ml Formamid werden während 18 Stunden bei einer Temperatur von 110° C gerührt. Dann wird die tiefgrüne Lösung auf Raumtemperatur gekühlt, mit 30 ml Ethanol verdünnt, der Farbstoff als Kalium-Salz durch Zugabe von 5.0 g Kaliumacetat ausgefällt und der Niederschlag abfiltriert. Man erhält so 4.0 g des Zink-Phthalocyanin-Farbstoffs (100) in der Form seines Kalium-Salzes.

Anstelle von 1,8-Diazabicyclo(4.4.0)undec-7-en kann auch eine andere starke organische Base wie beispielsweise 1,4-Diazabicyclo(2.2.2)octan verwendet werden. Anstelle von Kaliumacetat kann auch das Acetat oder das Chlorid eines anderen Alkalimetalls, wie beispielsweise Natriumacetat oder Lithiumchlorid, verwendet werden, wobei dann natürlich der Zink-Phthalocyanin-Farbstoff in Form des Natrium- beziehungsweise Lithium-Salzes anfällt.

Auf ähnliche Art und Weise können durch die Wahl geeigneter Mengen anderer Ausgangsmaterialien die erfindungsgemässen symmetrischen Farbstoffe (101) bis (106) hergestellt werden.

### Beispiel 2 (Farbstoff des Stands der Technik)

Ein asymmetrischer Zink-Phthalocyanin-Farbstoff wurde folgendermassen hergestellt:
7.7 g (30 mMol) des Anilins der Formel (X), hergestellt nach dem von G. P. Britovsek, G. Y. Y. Woo und N. Assavathorn in "Synthesis and reactivity of watersoluble platinum(II) complexes containing nitrogen ligands", Journal of Organometallic Chemistry 679, 110 - 115 (2003) beschriebenen Verfahren, 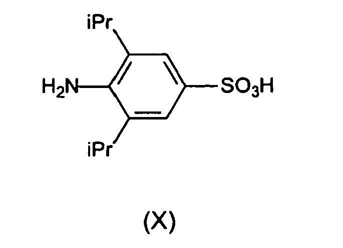 werden in 30 ml Pyridin suspendiert.

Zu dieser Suspension werden unter Rühren während 10 Minuten 5.7 g (30 mMol) des Säurechlorids der Formel (VIII), gelöst in 8 ml Ethylacetat, unter Kühlung mit Eis so zugetropft, dass die Temperatur 30° C nicht übersteigt. Nach Ende der Zugabe wird der Ansatz 18 Stunden bei Raumtemperatur nachgerührt. Anschliessend wird das Gemisch mit 20 ml N-Methylpyrrolidon und 20 ml Ethylacetat verdünnt, klarfiltriert, und das Filtrat eingeengt. Das Rohprodukt wird durch Verrühren mit 50 ml gesättigter Kochsalzlösung gereinigt. Nach Trocknung erhält man 6.7 g des Amids der Formel (XI)

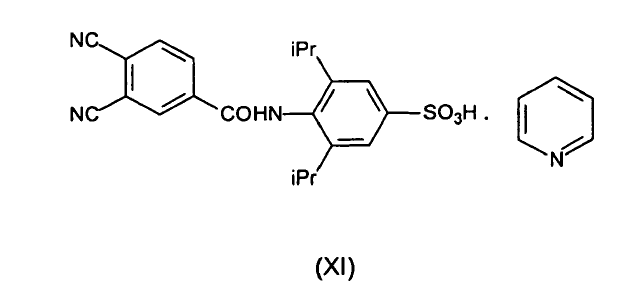

3.5 g (8 mMol) des Amids der Formel (IX) und 3.9 g (8 mMol) des Amids der Formel (XI), 0.88 g (4 mmol) Zinkacetat-Dihydrat und 2.4 g 1,8-Diazabicyclo(5.4.0) undec-7-en in 10 ml Formamid werden während 18 Stunden bei einer Temperatur von 110° C gerührt. Dann wird die tiefgrüne Lösung auf Raumtemperatur gekühlt, mit 30 ml Ethanol verdünnt, der Farbstoff als Kalium-Salz durch Zugabe von 5 g Kaliumacetat ausgefällt und der Niederschlag abfiltriert. Man erhält so 3.0 g eines Zink-Phthalocyanin-Farbstoffgemisches in Form seines Kalium-Salzes.

Dieses Zink-Phthalocyanin-Farbstoffgemisch, enthält unter anderen den Zink-Phthalocyanin-Farbstoff der Formel 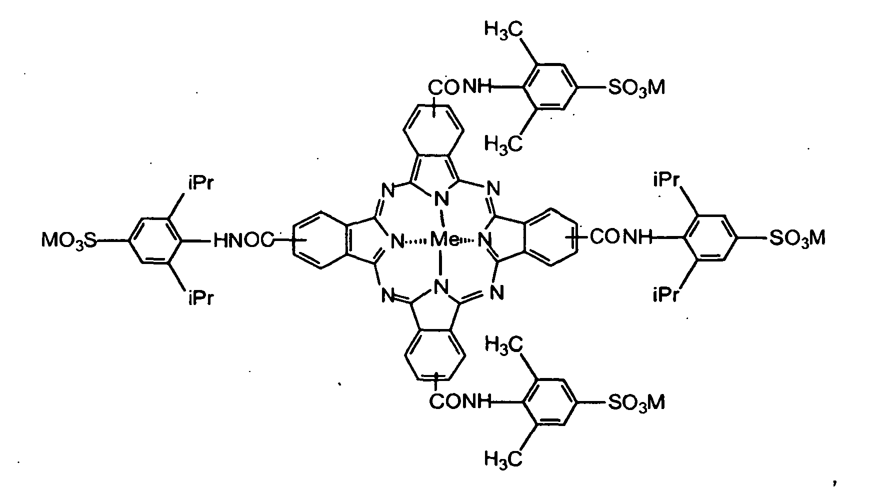 den Zink-Phthalocyanin-Farbstoff der Formel 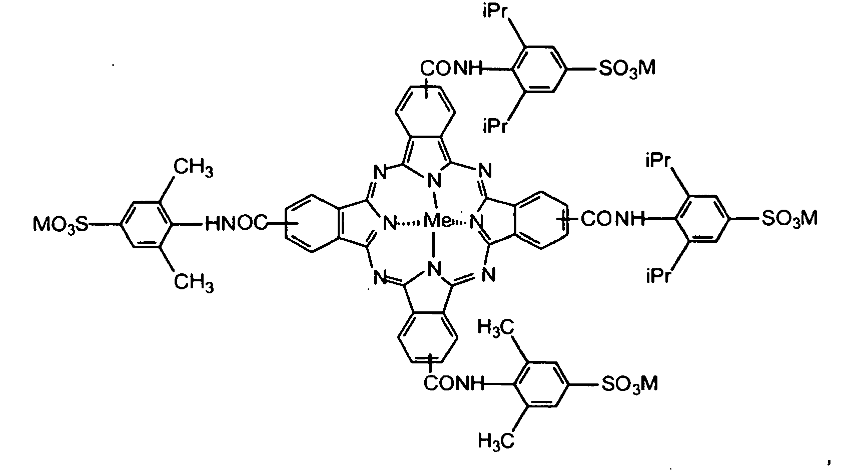 den Zink-Phthalocyanin-Farbstoff der Formel 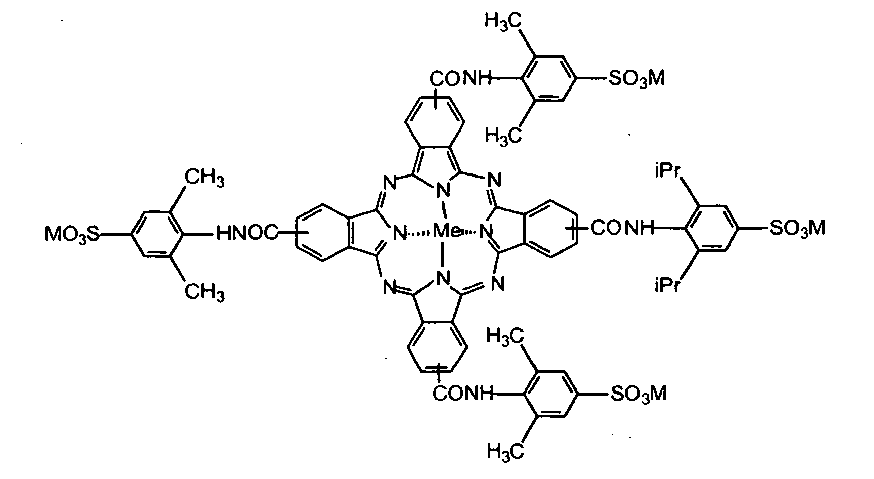 und den Zink-Phthalocyanin-Farbstoff der Formel

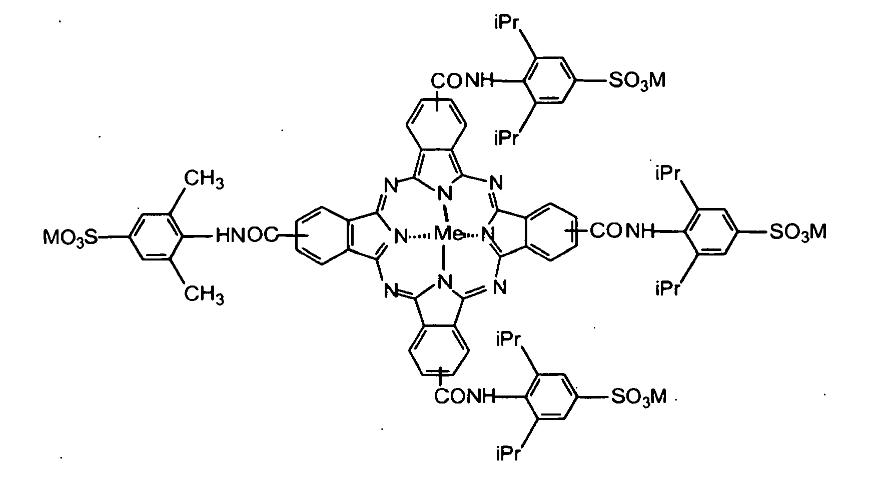

In den vorangehenden 4 Formeln steht Me für Zink.

### Prüfungen

### 1. Lichtabsorption in wässriger Lösung

Das Absorptionsspektrum wurde bei einer Konzentration von 20 bis 30 mg/l des Phthalocyanin-Farbstoffs in einer Zelle mit einer Dicke von 1 cm mit einem Spektrometer Cary 100 Bio UV/VIS (erhältlich bei Varian Inc., Palo Alto, USA) in einer gepufferten wässrigen Lösung, welche 50 Gewichtsprozent Polyethylenglykol 400 enthält, bei einem pH-Wert von 7.0 gemessen.

In wässriger Lösung aggregieren die Phthalocyanin-Farbstoffe. Das Absorptionsmaximum ist deshalb dasjenige des gebildeten Aggregats.

### 2. Lichtabsorption in einer wässrigen Polyethylenglykol-Lösung

Das Absorptionsspektrum wurde bei einer Konzentration von 20 bis 30 mg/l des Phthalocyanin-Farbstoffs in einer Zelle mit einer Dicke von 1 cm mit einem Spektrometer Cary 100 Bio UV/VIS in einer gepufferten wässrigen Lösung bei einem pH-Wert von 7.0 gemessen.

In der wässrigen Lösung von Polyethylenglykol aggregieren die Phthalocyanin-Farbstoffe kaum. Das Absorptionsmaximum ist deshalb dasjenige des monomeren Phthalocyanin-Farbstoffs. Dieses Absorptionsmaximum liegt bei höheren Wellenlängen als das Absorptionsmaximums des Phthalocyanin-Farbstoffaggregats.

### 3. Lichtabsorption in Gegenwart von fötalem Kälberserum

Das Absorptionsspektrum wurde mit einem Spektrometer Cary 100 Bio UV/VIS zwischen 300 nm und 800 nm gemessen. 1 mg Phthalocyanin-Farbstoff wurde in 1 ml einer Lösung von Polyethylenglykol 400, Ethanol und Wasser (30 : 20 : 50) gelöst und die erhaltene Lösung wurde mit physiologischer Kochsalzlösung, welche zusätzlich noch fötales Kälberserum in einer Konzentration von 10 % aufwies, auf eine Konzentration von 10 µg / ml verdünnt. Diese Lösung, welche fötales Kälberserum enthält, simuliert die biologischen Bedingungen im Zellinnern.

Für die dynamische Phototherapie besonders geeignete Phthalocyanin-Farbstoffe zeigen in der Lösung, welche das fötale Kälberserum enthält, ein möglichst hohes Verhältnis der Absorption im Absorptionsmaximum der monomeren Form zur Absorption im Absorptionsmaximum der aggregierten Form des Phthalocyanin-Farbstoffs.

### 4. Photodynamische Aktivität

Die Phthalocyanin-Farbstoffe wurden nach dem von L. Bourré, G. Simonneaux, Y. Ferrand, S. Thibaut, Y. Lajat und T. Patrice in "Synthesis, and in vitro and in vivo evaluation of a diphenylchlorin sensitizer for photodynamic therapy", Journal of Photochemistry and Photobiology B: Biology 69, 179 - 192 (2003) angegebenen Verfahren auf ihre photodynamische Aktivität geprüft.

Dabei wurden Zellen der gegen eine photodynamische Therapie besonders resistenten Zelllinien HT29 (menschliche Adenocarcinom-Zellen, ECACC Nr. 91072201, erhältlich bei CERDIC, Sophia-Antipolis, Frankreich) und F98 im Zellkulturmedium RPMI 1640 nach dem einem Fachmann bekannten Verfahren (Abschnitt 2.2.3 des vorhin zitierten Artikels) für die photodynamische Therapie vorbereitet. Nach 24 Stunden wurden die Zellen mit dem Phthalocyanin-Farbstoff bei Konzentrationen bis zu 10 µg / ml während 4 Stunden inkubiert. Anschliessend wurden die Zellen mit einem abstimmbaren Laser CR 599 (erhältlich bei Coherent Laser Group, Santa Clara, USA) über eine Lichtleitfaser mit einer Strahlungsmenge von 20 J / cm² bestrahlt. Die Menge der die photodynamische Therapie überlebenden Zellen wurden mittels MMT-Kolorimetrie mit [3-(4,5-Dimethlylthiazol-2-yl)-2,5-diphenyltetrazoliumbromid (erhältlich bei Sigma-Aldrich Chemie GmbH, Buchs, Schweiz) bestimmt. Die verwendete Strahlungsmenge entspricht etwa der in der photodynamischen Therapie von menschlichem Gewebe in vivo normalerweise eingesetzten Strahlungsmenge von 200 J / cm².

### 5. Reaktive Sauerstoff-Spezies

Die Menge der unter Lichteinfluss entstehenden reaktiven Sauerstoff-Spezies wurde folgendermassen bestimmt:
Der zu prüfende Phthalocyanin-Farbstoff wurde in einer wässrigen (10 %) Lösung von fötalem Kälberserum in einer Konzentration von 10 µg / ml gelöst. Luminol (3-Phthalhydrazid, erhältlich bei Sigma-Aldrich Chemie GmbH, Buchs, Schweiz) wurde in einer Konzentration von 0.5 mg / ml in einer 0.1 molaren Phosphatpuffer-Lösung bei einem pH-Wert von 12.4 gelöst. 1 ml der Lösung von Luminol wurde in eine quadratische Spektrofluorometerküvette mit einer Seitenlänge von 1 cm gegeben, welche die Lösung des zu prüfenden Phthalocyanin-Farbstoffs enthielt. Anschliessend wurde die Küvette bei einer Temperatur von 30 °C mit insgesamt 10 J / m² Licht im Absorptionsmaximum des zu prüfenden Phthalocyanin-Farbstoffs bestrahlt. Dabei entstanden die reaktiven Sauerstoff-Spezies, welche das Luminol zu fluoreszierender Aminophthalsäure mit einem Absorptionsmaximum bei 485 nm oxidierten. Das Fluoreszenzspektrum dieser Lösung wurde mit einem Fluoreszenzspektrometer Cary PCB 150 (erhältlich bei Varian Inc., Palo Alto, USA) 15 Minuten nach beendeter Bestrahlung gemessen, nachdem die Aminophthalsäure mit Licht der Wellenlänge 395 nm angeregt worden war. Die Menge der entstandenen reaktiven Sauerstoff-Spezies ist proportional zum Unterschied der gemessenen Fluoreszenz der bestrahlten Testküvette und einer bestrahlten Vergleichsküvette, welche keinen zu prüfenden Phthalocyanin-Farbstoff enthielt. Die reaktivsten Phthalocyanin-Farbstoffe weisen den grössten Zahlenwert (in willkürlichen Einheiten) auf.

### Kurze Beschreibung der Zeichnung

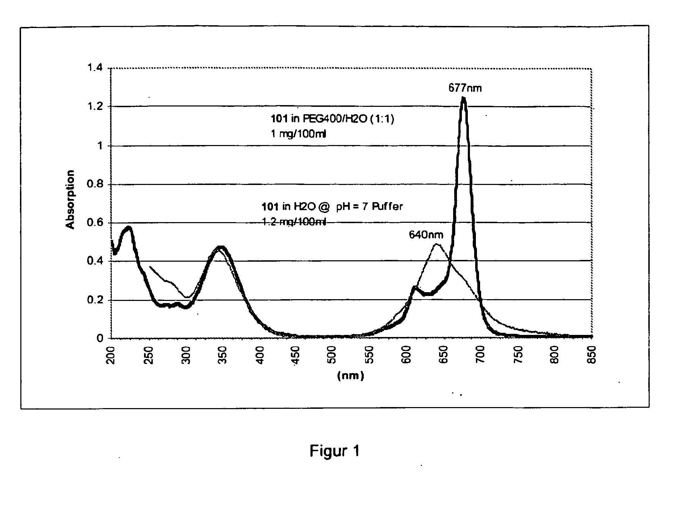

Die normierten Absorptionsspektren des Farbstoffs (100) in wässriger Lösung (Absorptionsmaximum bei 640 nm) und in einer wässrigen Polyethylenglykol-Lösung (Absorptionsmaximum bei 677 nm) sind in Figur 1 aufgezeichnet.

### Ergebnisse

Die gemessenen Absorptionsmaxima der Phthalocyanin-Farbstoffe mit verschiedenen Metallen Me und verschiedenen M-Ionen sowie dem bekannten Phthalocyanin-Farbstoff der Formel (I) sind in Tabelle 1 zusammengestellt.

**Tabelle 1**

| Farbstoff Nr. | Me | M | λₘₐₓ in H₂O | λₘₐₓ in H₂O / PEG 400 |
|---|---|---|---|---|
| | | | (nm) | (nm) |
| 100 (Stand der Technik) | Zn | K | 628 | 687 |
| 101 (Stand der Technik) | Zn | Na | 638 | 685 |
| 101 (Stand der Technik) | Al | K | 694 | 683 |
| 101 | Mg | K | 641 | 688 |
| 101 | H | K | 608 | 639, 677 |
| 102 (Stand der Technik) | Zn | K | 638 | 685 |
| 103 (Stand der Technik) | Zn | K | 623 | 685 |
| 104 (Stand der Technik) | Zn | K | 625 | 685 |
| 105 (Stand der Technik) | Zn | K | 633 | 685 |
| 106 (Stand der Technik) | Zn | Li | 681 | 685 |
| (I) | Zn | H | 611 | 678 |

Das Absorptionsmaximum des Farbstoff (106) wurde in einer 1:1- Mischung von Wasser und Ethanol gemessen.

Der Phthalocyanin-Farbstoff der Formel (I) ist erhältlich bei Frontier Scientific, Logan, USA).

Ein Vergleich der Absorptionsmaxima in den beiden Lösungen in Tabelle 1 zeigt klar, dass der erfindungsgemässe Phthalocyanin-Farbstoffe (101) sowie die Phthalocyanin-Farbstoffe (100) bis (106), bei denen Me für Aluminium oder Zink steht, und der Phthalocyanin-Farbstoff der Formel (I) des Stands der Technik in wässriger Lösung vor allem als Aggregate vorliegen und in der wässrigen Polethylenglykol-Lösung in ihrer monomeren Form.

Die Ergebnisse der Messung der Lichtabsorption in Gegenwart von fötalem Kälberserum des Zink-Phthalocyanin-Farbstoffs (101) und des Phthalocyanin-Farbstoffs der Formel (I) des Stands der Technik, je in einer Konzentration von 10 µg / ml, sind in Tabelle 2 zusammengestellt. Angegeben wird Verhältnis der Absorption im Absorptionsmaximum der monomeren Form zur Absorption im Absorptionsmaximum der aggregierten Form des Phthalocyanin-Farbstoffs.

**Tabelle 2**

| Farbstoff | Absorptionsverhältnis |
|---|---|
| 101 | 0.79 |
| I | 0.58 |

Ein Vergleich der Werte in Tabelle 2 zeigt klar, dass der Zink-Phthalocyanin-Farbstoff (101) ein für die photodynamische Behandlung geeigneteres Verhältnis der Absorption im Absorptionsmaximum der monomeren Form zur Absorption im Absorptionsmaximum der aggregierten Form hat als der Phthalocyanin-Farbstoff der Formel (I) des Stands der Technik.

Die Ergebnisse der photodynamischen Aktivitätsprüfung bei einer Bestrahlung im Absorptionsmaximum der monomeren Form des Zink-Phthalocyanin-Farbstoffs (101) und des Phthalocyanin-Farbstoffs der Formel (I) des Stands der Technik, je in einer Konzentration von 10 µg / ml, sind in Tabelle 3 zusammengestellt.

**Tabelle 3**

| Farbstoff | Zelllinie | Anzahl überlebende Zellen |
|---|---|---|
| 101 | HT 29 | 12% |
| I | HT 29 | 70% |
| 101 | F 98 | 18 % |
| I | F98 | 84% |

Die Ergebnisse aus Tabelle 3 zeigen sofort, dass es beim Zink-Phthalocyanin-Farbstoff (101) bei beiden Zelllinien wesentlich weniger überlebende Zellen hat als beim Phthalocyanin-Farbstoff der Formel (I) des Stands der Technik. Der Zink-Phthalocyanin-Farbstoff (101) hat somit eine wesentlich höhere photodynamische Aktivität als der Phthatocyanin-Farbstoff der Formel (I) des Stands der Technik.

Die Ergebnisse der Bestimmung der Menge der reaktiven Sauerstoff-Spezies für den Zink-Phthalocyanin-Farbstoff (101) und den Phthalocyanin-Farbstoff der Formel (I) des Stands der Technik sind in Tabelle 4 zusammengestellt.

**Tabelle 4**

| Farbstoff | Bestrahlungswellenlänge | Menge der reaktiven Sauerstoff- |
|---|---|---|
| | (nm) | Spezies (willkürliche Einheiten) |
| 101 | 632 | 4.71 |
| 101 | 683 | 5.12 |
| (I) | 675 | 3.51 |

Die Ergebnisse aus Tabelle 4 zeigen sofort, dass der erfindungsgemässe Zink-Phthalocyanin-Farbstoff (101) wesentlich mehr reaktive Sauerstoff-Spezies erzeugt als der Phthalocyanin-Farbstoff der Formel (I) des Stands der Technik, sogar bei Bestrahlung im Absorptionsmaximum der aggregierten Form des Phthalocyanin-Farbstoffs (101).

## Patentansprüche

1. Phthalocyanin-Farbstoffe der allgemeinen Formel (II) 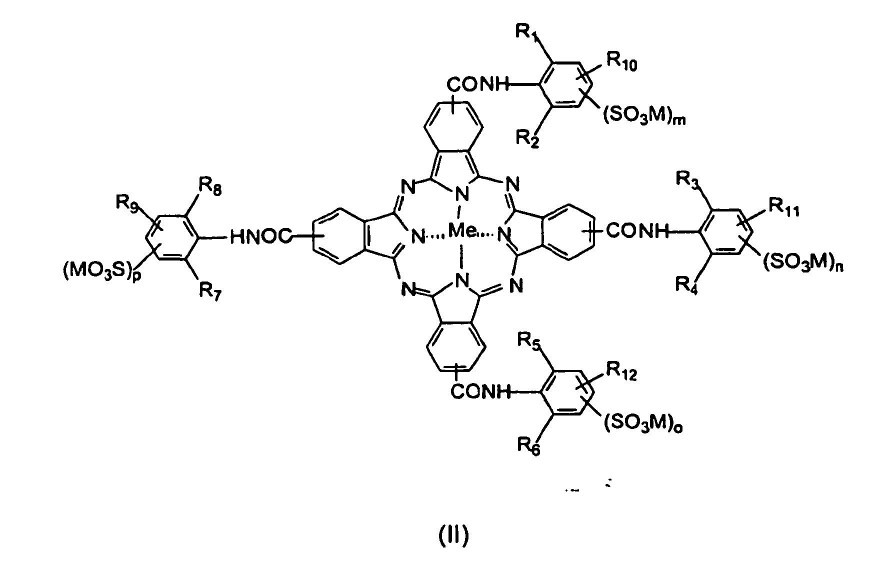 worin
R₁ - R₈ unabhängig voneinander für einen linearen oder einen verzweig- ten Alkylrest mit bis zu 8 Kohlenstoffatomen;
**R₉ -** R₁₂ unabhängig voneinander für Wasserstoff, einen linearen oder ei- nen verzweigten Alkylrest mit bis zu 8 Kohlenstoffatomen oder ein Halogenatom;
Me für Magnesium, Wasserstoff, Gallium, Germanium oder Zinn;
M für Wasserstoff, ein Metallkation oder ein Ammoniumkation, das gegebenenfalls einen oder mehrere unsubstituierte oder substitu- ierte Alkylreste oder Hydroxyalkoxyalkylreste mit jeweils 1 bis 12 Kohlenstoffatomen aufweist, stehen
und
m, n, o, p unabhängig voneinander den Wert 1 oder 2 annehmen.

2. Phthalocyanin-Farbstoffe gemäss Anspruch 1, **dadurch gekennzeichnet, dass** R₁ - R₈, R₉ **-** R₁₂, Me und M die gleiche Bedeutung wie in Anspruch 1 haben
und
m, n , o, p den Wert 1 annehmen.

3. Phthalocyanin-Farbstoffe der allgemeinen Formel (III) gemäss Anspruch 1, 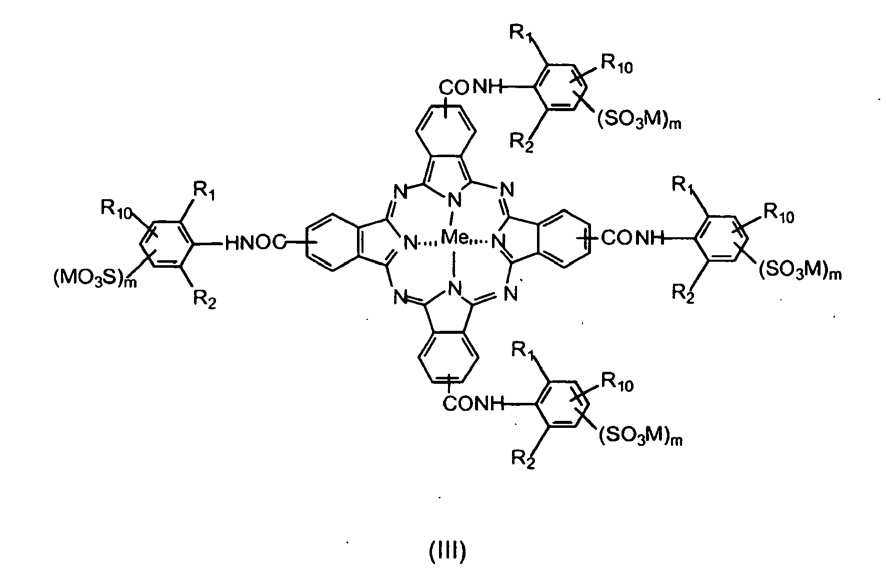 worin R₁, R₂, R₁₀, Me, M und m die gleiche Bedeutung wie in Anspruch 1 haben.

4. Phthalocyanin-Farbstoffe gemäss Anspruch 3, **dadurch gekennzeichnet, dass** R₁, R₂, R₁₀, Me und M die gleiche Bedeutung wie in Anspruch 1 haben
und
m den Wert 1 annimmt.

5. Verfahren zur Herstellung der Phthalocyanin-Farbstoffe nach Anspruch 1, **dadurch gekennzeichnet, dass**
ein aromatisches Amin der allgemeinen Formel (IV A), 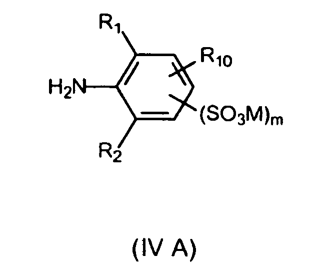 worin R₁, R₂, R₁₀ und m die gleiche Bedeutung wie in Anspruch 1 haben,
der allgemeinen Formel (IV B), 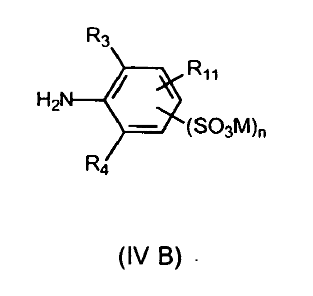 worin R₃, R₄, R₁₁ und n die gleiche Bedeutung wie in Anspruch 1 haben,
der allgemeinen Formel (IV C), 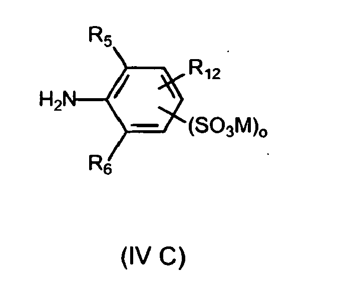 worin R₅, R₆, R₁₂ und o die gleiche Bedeutung wie in Anspruch 1 haben,
und
der allgemeinen Formel (IV D), 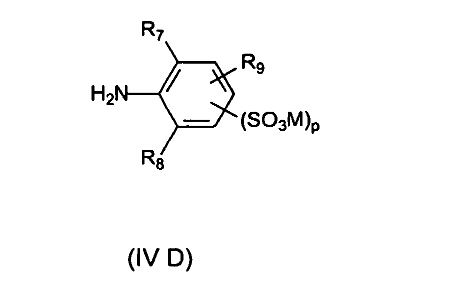 worin R₇, R₈, **R₉** und p die gleiche Bedeutung wie in Anspruch 1 haben,
mit dem Carbonsäurechlorid der Formel (V) 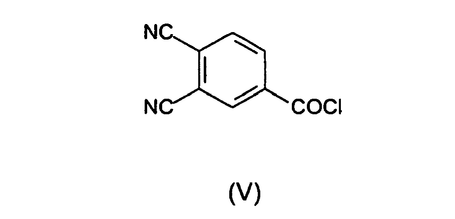 unter Bedingungen umgesetzt werden, dass die Amide der allgemeinen Formeln 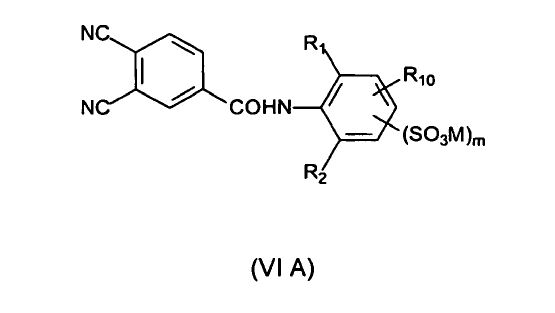 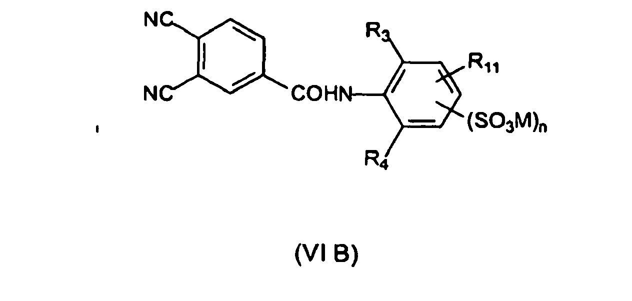 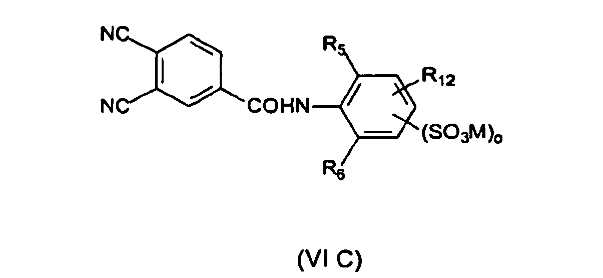 und 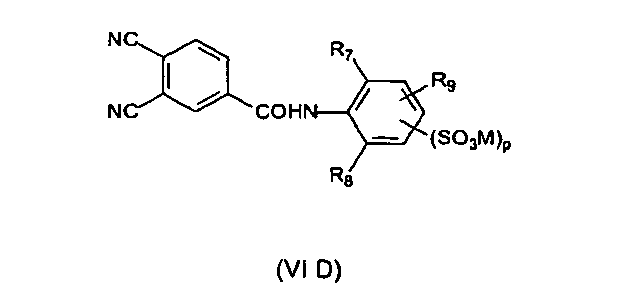 gebildet werden,
und
ein Gemisch der Amide der allgemeinen Formeln (VI A), (VI B), (VI C) und (VI D) mit einem Salz des Metalls Me, falls Me nicht für Wasserstoff steht, umgesetzt wird.

6. Pharmazeutische Zusammensetzungen, welche einen oder mehrere Phthalocyanin-Farbstoffe der allgemeinen Formel (II),
worin m, n, o, p, R₁ - R₈, R₉ - R₁₂ und M die gleiche Bedeutung wie in Anspruch 1 haben
und
Me für Zink, Aluminium, Magnesium, Wasserstoff, Gallium, Germanium oder Zinn steht,
zusammen mit einer pharmazeutischen Trägersubstanz enthalten.

7. Pharmazeutische Zusammensetzungen, welche einen oder mehrere Phthalocyanin-Farbstoffe der allgemeinen Formel (II),
worin R₁ - R₈, R₉ - R₁₂ und M die gleiche Bedeutung wie in Anspruch 1 haben,
m, n, o, p die gleiche Bedeutung wie in Anspruch 2 haben
und
Me für Zink, Aluminium, Magnesium, Wasserstoff, Gallium, Germanium oder Zinn steht,
zusammen mit einer pharmazeutischen Trägersubstanz enthalten.

8. Pharmazeutische Zusammensetzungen, welche einen oder mehrere Phthalocyanin-Farbstoffe der allgemeinen Formel (III),
worin m, R₁, R₂, R₁₀ und M die gleiche Bedeutung wie in Anspruch 1 haben und
Me für Zink, Aluminium, Magnesium, Wasserstoff, Gallium, Germanium oder Zinn steht,
zusammen mit einer pharmazeutischen Trägersubstanz enthalten.

9. Pharmazeutische Zusammensetzungen gemäss Anspruch 8, **dadurch gekennzeichnet, dass**
m den Wert 1 annimmt.

10. Pharmazeutische Zusammensetzungen gemäss den Ansprüchen 6 bis 9, **dadurch gekennzeichnet, dass** die pharmazeutische Trägersubstanz ein Gemisch von Polyethylenglykol, Ethanol und Wasser ist oder aus Liposomen, Nanocontainem oder Biopolymeren oder deren Gemischen besteht oder solche enthält.

11. Verwendung der pharmazeutischen Zusammensetzungen gemäss den Ansprtichen 6 bis 10 als Arzneimittel in der photodynamischen Therapie oder als photodiagnostische Reagenzien.

12. Verwendung der pharmazeutischen Zusammensetzungen gemäss den Ansprüchen 6 bis 10 zur Behandlung von durch Viren, Pilzen oder Bakterien ausgelösten Infektionskrankheiten, Krebs und dermatologischen Erkrankungen.

13. Verwendung der Phthalocyanin-Farbstoffe gemäss den Ansprüchen 1 bis 4 sowie der Phthalocyanin-Farbstoffe der allgemeinen Formel (II),
worin m, n, o, p, R₁ - R₈, R₉ - R₁₂ und M die gleiche Bedeutung wie in Anspruch 1 haben;
Me für Zink, Aluminium, Magnesium, Wasserstoff, Gallium, Germanium oder Zinn steht;
sowie der Phthalocyanin-Farbstoffe der allgemeinen Formel (III),
worin m, R₁, R₂, R₁₀ und M die gleiche Bedeutung wie in Anspruch 1 haben und
Me für Zink, Aluminium, Magnesium, Wasserstoff, Gallium, Germanium oder Zinn steht,
in der photodynamischen Therapie oder als photodiagnostische Reagenzien und zur Behandlung von durch Viren, Pilzen oder Bakterien ausgelösten Infektionskrankheiten, Krebs und dermatologischen Erkrankungen.

## Claims

1. Phthalocyanine dyes of general formula (II) 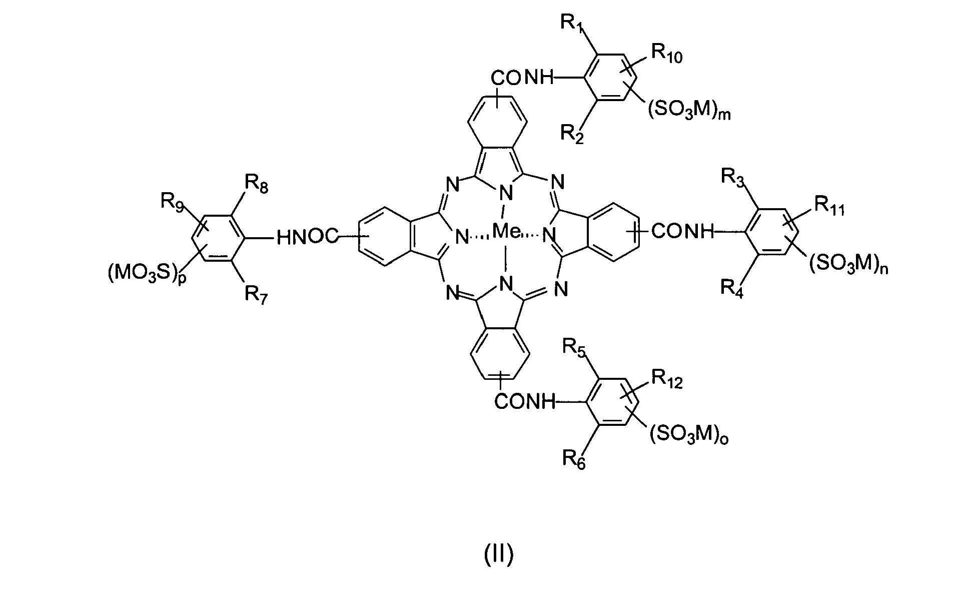 wherein
R₁ - R₈ independently represent a linear or branched alkyl group having up to 8 carbon atoms;
R₉ - R₁₂ independently represent a hydrogen atom, a linear or branched al- kyl group having up to 8 carbon atoms or a halogen atom;
Me represents a magnesium atom, a hydrogen atom, a gallium atom, a germanium atom or a tin atom;
M represents a hydrogen atom, a metal cation or an ammonium cation, which optionally may be substituted by one or more unsub- stituted or substituted alkyl or hydroxyalkoxyalkyl groups each having from 1 to 12 carbon atoms;
and
m, n , o, p independently are 1 or 2.

2. Phthalocyanine dyes according to claim 1, **characterised by** the fact that
R₁ - R₈, R₉ - R₁₂, Me and M are as defined in claim 1
and
m, n , o, p are equal to 1.

3. Phthalocyanine dyes of general formula (III) according to claim 1, 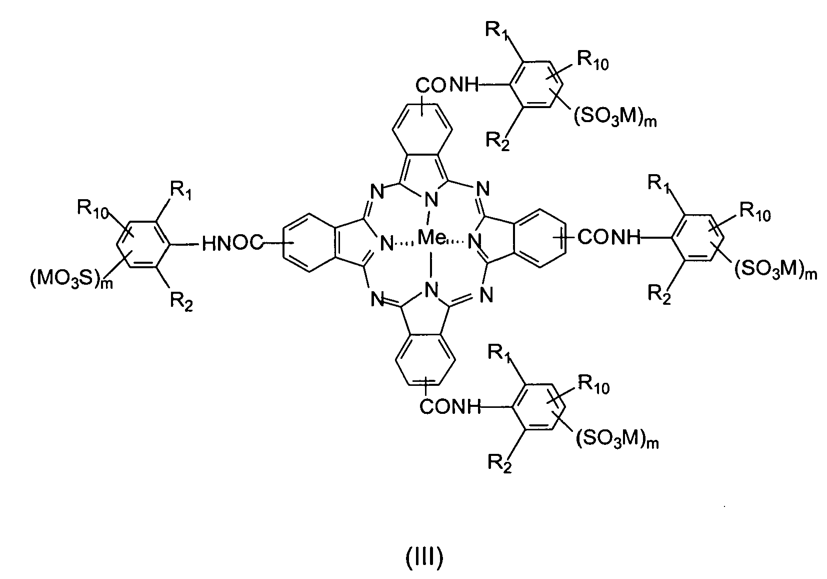 **characterised by** the fact that
R₁, R₂, R₁₀, Me, M and m are as defined in claim 1.

4. Phthalocyanine dyes according to claim 3, **characterised by** the fact that R₁, R₂, R₁₀, Me, M and m are as defined in claim 1
and
m is equal to 1.

5. Method of preparation of the phthalocyanine dyes according to claim 1, **characterised by** the fact that
an aromatic amine of general formula (IV A), 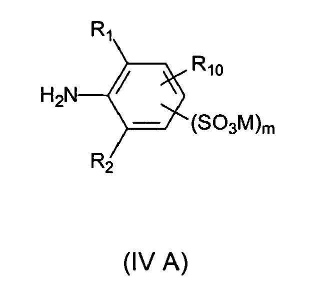 wherein R₁, R₂, R₁₀ and m are as defined in claim 1;
of general formula (IV B), 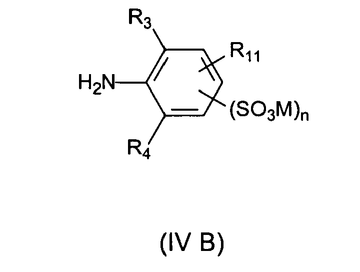 wherein R₃, R₄, R₁₁ and n are as defined in claim 1;
of general formula (IV C), 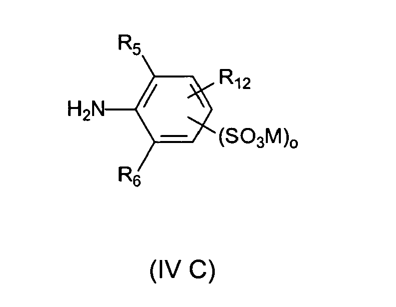 wherein R₅, R₆, R₁₂ and o are as defined in claim 1;
and
of general formula (IV D), 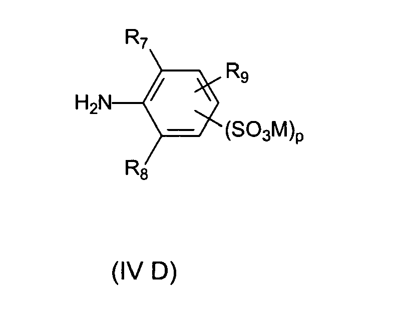 wherein R₇, R₈, R₉ and p are as defined in claim 1,
is reacted with the acyl chloride of formula (V) 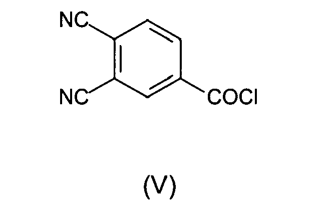 under conditions that the amides of general formulas 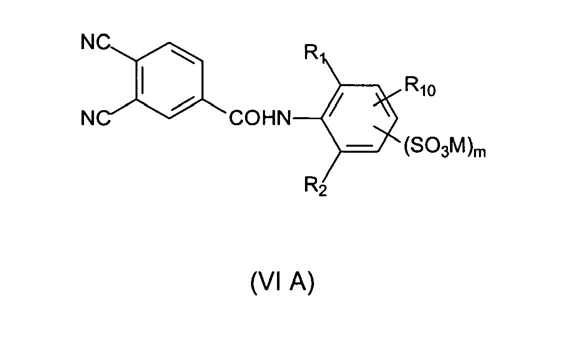 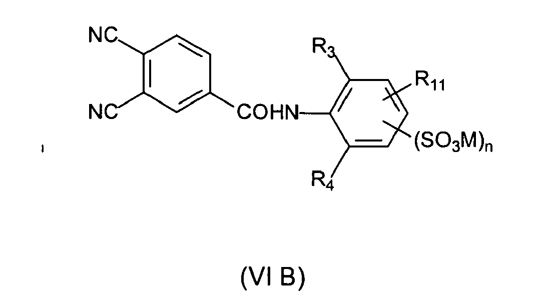 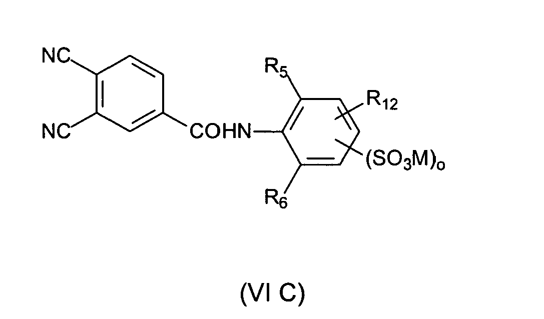 and 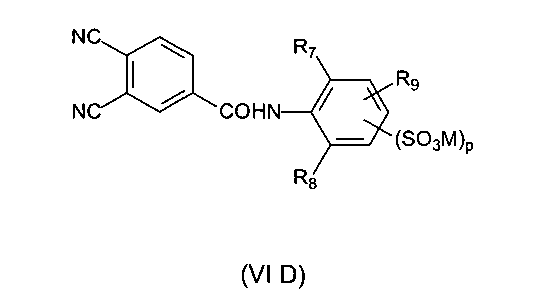 are formed,
and
a mixture of the amides of general formulas (VI A), (VI B), (VI C) and (VI D) is reacted with a salt of metal Me, if Me does not represent a hydrogen atom.

6. Pharmaceutical compositions comprising a pharmaceutical carrier and one or more phthalocyanine dyes according to claim 1, **characterised by** the fact that
m, n, o, p, R₁ - R₈, R₉ - R₁₂ and M are as defined in claim 1
and
Me represents a zinc atom, an aluminium atom, a magnesium atom, a hydrogen atom, a gallium atom, a germanium atom or a tin atom.

7. Pharmaceutical compositions comprising a pharmaceutical carrier and one or more phthalocyanine dyes of general formula (II), **characterised by** the fact that
R₁ - R₈, R₉ - R₁₂ and M are as defined in claim 1;
m,n, o and p are as defined in claim 2
and
Me represents a zinc atom, an aluminium atom, a magnesium atom, a hydrogen atom, a gallium atom, a germanium atom or a tin atom.

8. Pharmaceutical compositions comprising a pharmaceutical carrier and one or more phthalocyanine dyes of general formula (III), **characterised by** the fact that
m, R₁, R₂, R₁₀ and M are as defined in claim 1
and
Me represents a zinc atom, an aluminium atom, a magnesium atom, a hydrogen atom, a gallium atom, a germanium atom or a tin atom.

9. Pharmaceutical compositions according to claim 8, **characterised by** the fact that
m is equal to 1.

10. Pharmaceutical compositions according to claims 6 to 9, **characterised by** the fact that
the pharmaceutical carrier is a mixture of polyethylene glycol, ethanol and water or consists of or contains liposomes, nano containers, biopolymers or a mixture thereof.

11. Use of the pharmaceutical compositions according to claims 6 to 10 as medicaments for photodynamic therapy or as photodiagnostic agents.

12. Use of the pharmaceutical compositions according to claims 6 to 10 as medicaments for the treatment of viral, fungal or viral infection diseases, cancer or dermatological diseases.

13. Use of the phthalocyanine dyes according to claims 1 to 4 and of general formula (II), **characterised by** the fact that
m, n, o, p, R₁ - R₈, R₉ - R₁₂ and M are as defined in claim 1;
Me represents a zinc atom, an aluminium atom, a magnesium atom, a hydrogen atom, a gallium atom, a germanium atom or a tin atom
and
of general formula (III), **characterised by** the fact that m, R₁, R₂, R₁₀ and M are as defined in claim 1 and
Me represents a zinc atom, an aluminium atom, a magnesium atom, a hydrogen atom, a gallium atom, a germanium atom or a tin atom,
in photodynamic therapy or as photodiagnostic agents or for the treatment of viral, fungal or viral infection diseases, cancer or dermatological diseases.

## Revendications

1. Colorants phthalocyanines de formule générale (II) 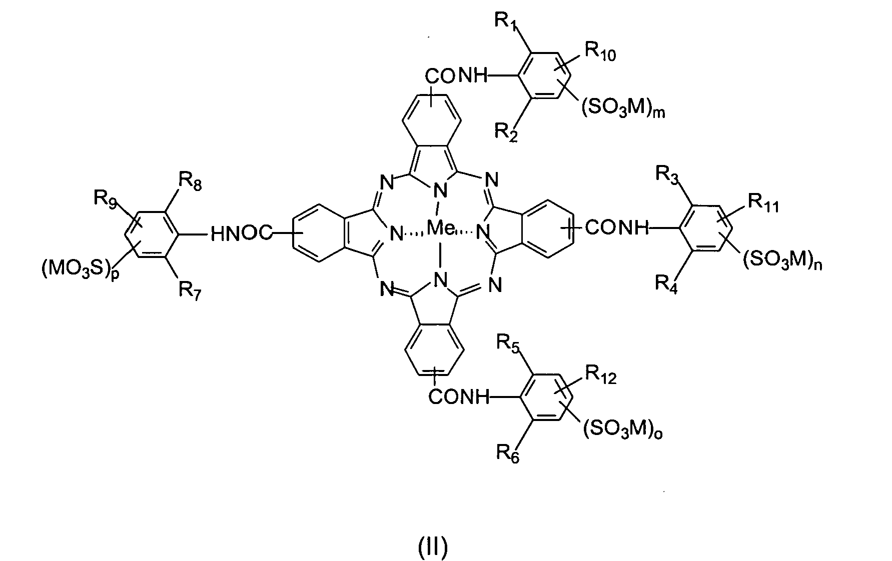 dans laquelle
R₁ - R₈ représentent indépendamment un radical alkyle branché ou non- branché ayant jusqu'à 8 atomes de carbone;
R₉ - R₁₂ représentent indépendamment un atome d'hydrogène, un radical alkyle branché ou non-branché ayant jusqu'à 8 atomes de car- bone ou un atome d'halogène;
Me représente un atome de magnésium, un atome d'hydrogène, un atome de gallium, un atome de germanium ou un atome d'étain;
M représente un atome d'hydrogène, un cation métallique ou un ca- tion ammonium optionnellement substitué par un ou plusieurs ra- dicaux alkyle ou alkyle substitué ou hydroxyalcoxyalkyle ayant chacun de 1 à 12 atomes de carbone;
et
m, n, o, p valent indépendamment 1 ou 2.

2. Colorants phthalocyanines selon la revendication 1, **caractérisés par le fait que**
R₁ - R₈, R₉ - R₁₂, Me et M sont définis comme dans la revendication 1
et
m, n , o, p valent 1.

3. Colorants phthalocyanines de formule générale (III) selon la revendication 1,
**caractérisés par le fait que** 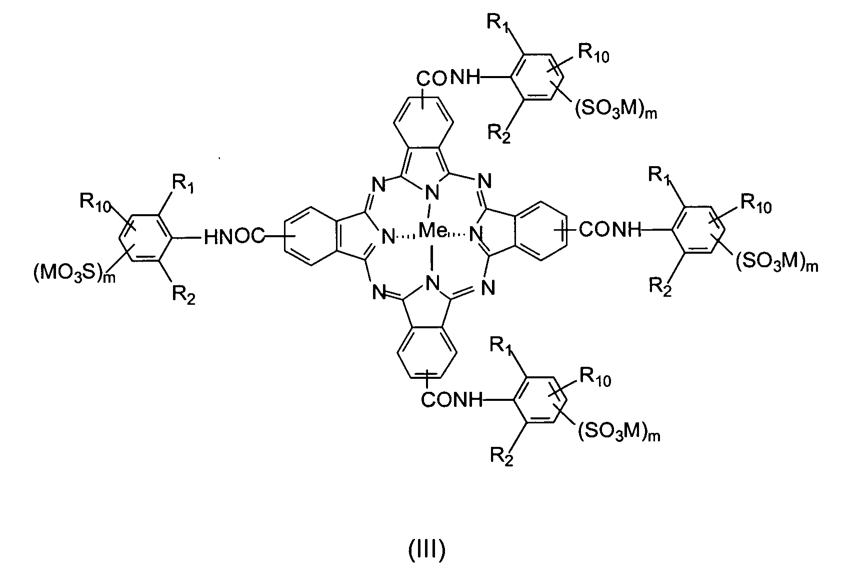 dans laquelle
R₁, R₂, R₁₀, Me, M et m sont définis comme dans la revendication 1.

4. Colorants phthalocyanines selon la revendication 3, **caractérisés par le fait que**
R₁, R₂, R₁₀, Me et M sont définis comme dans la revendication 1
et
m vaut 1.

5. Procédé de préparation des colorants phthalocyanines selon la revendication 1, **caractérisé par le fait que**
une amine aromatique de formule générale (IV A), 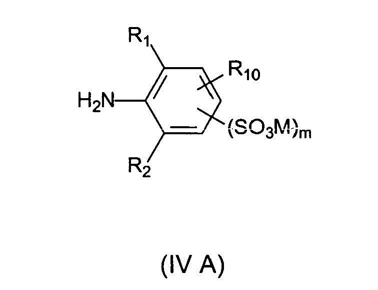 dans laquelle R₁, R₂, R₁₀ et m sont définis comme dans la revendication 1,
de formule générale (IV B), 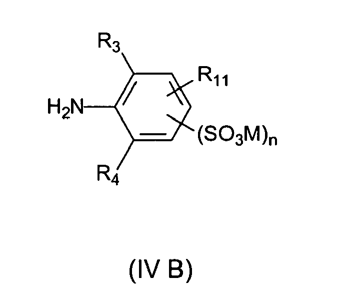 dans laquelle R₃, R₄, R₁₁ et n sont définis comme dans la revendication 1,
de formule générale (IV C), 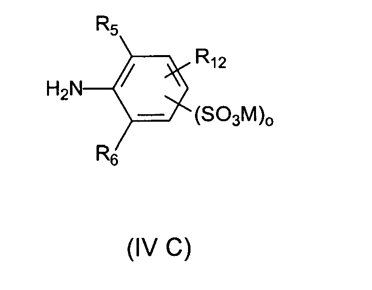 dans laquelle R₅, R₆, R₁₂ et o sont définis comme dans la revendication 1,
et
de formule générale (IV D), 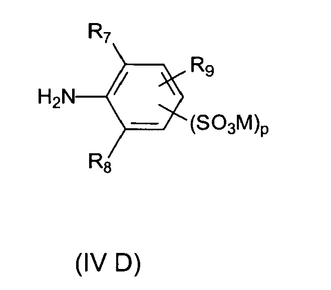 dans laquelle R₇, R₈, R₉ et p sont définis comme dans la revendication 1,
sont réagies avec le chlorure de l'acide carboxylique de formule (V) 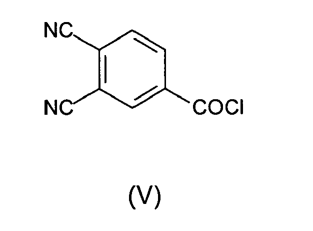 sous des conditions que les amides de formules générales 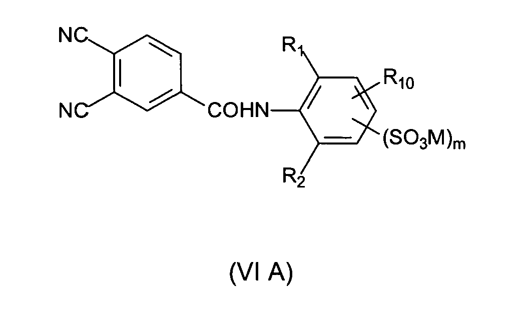 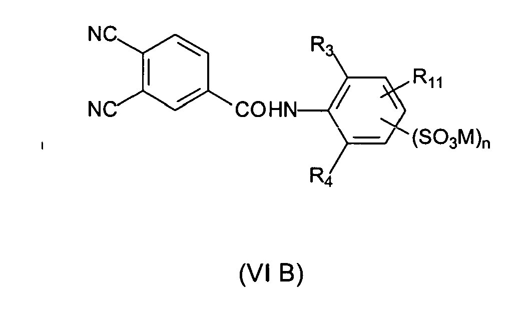 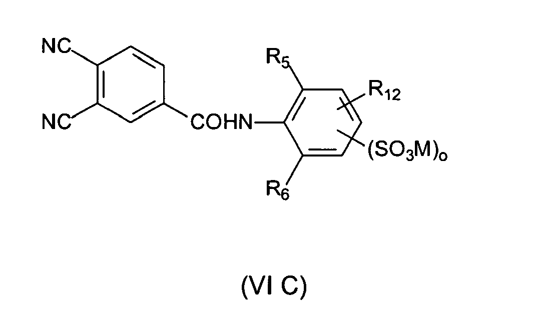 et 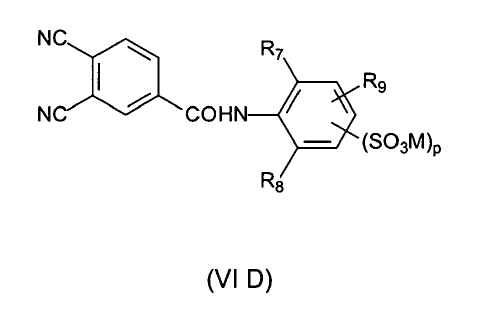 sont formés,
et
un mélange des amides de formules générales (VI A), (VI B), (VI C) et (VI D) ait réagi avec un sel du métal Me, si Me ne représente pas un atome d'hydrogène.

6. Formulations galéniques qui comportent conjointement un excipient pharmaceutique et un ou plusieurs colorants phthalocyanines de formule générale (II),
dans laquelle R₁ - R₈, R₉ - R₁₂ et M sont définis comme dans la revendication 1
et
Me représente un atome de zinc, un atome d'aluminium, un atome de magnésium, un atome d'hydrogène, un atome de gallium, un atome de germanium ou un atome d'étain.

7. Formulations galéniques qui comportent conjointement un excipient pharmaceutique et un ou plusieurs colorants phthalocyanines de formule générale (III),
dans laquelle R₁ - R₈, R₉ - R₁₂ et M sont définis comme dans la revendication 1,
m, n , o, p sont définis comme dans la revendication 2,
et
Me représente un atome de zinc, un atome d'aluminium, un atome de magnésium, un atome d'hydrogène, un atome de gallium, un atome de germanium ou un atome d'étain.

8. Formulations galéniques qui comportent conjointement un excipient pharmaceutique et un ou plusieurs colorants phthalocyanines de formule générale (III),
dans laquelle m, R₁, R₂, R₁₀ et M sont définis comme dans la revendication 1
et
Me représente un atome de zinc, un atome d'aluminium, un atome de magnésium, un atome d'hydrogène, un atome de gallium, un atome de germanium ou un atome d'étain.

9. Formulations galéniques selon la revendication 8, **caractérisées par le fait que**
m vaut 1.

10. Formulations galéniques selon les revendications 6 à 9, **caractérisées par le fait que**
l'excipient pharmaceutique est un mélange de polyéthylène glycol, d'éthanol et d'eau et contient des liposomes, des nanocontainers, des bio-polymères ou est constitué de liposomes, de nanocontainers, de bio-polymères ou leurs mélanges.

11. Utilisation des formulations galéniques selon les revendications 6 à 10 comme médicaments pour la thérapie photo-dynamique ou comme réactifs photo-diagnostiques.

12. Utilisation des formulations galéniques selon les revendications 6 à 10 pour le traitement de maladies infectueuses déclanchées par des virus, des champignons et des bactéries, du cancer et des maladies dermatologiques.

13. Utilisation des colorants phthalocyanines selon les revendications 1 à 4 ainsi que des colorants phthalocyanines de formules générales (II),
dans lesquelles
m, n, o, p, R₁ - R₈, R₉ - R₁₂ et M sont définis comme dans la revendication 1;
Me représente un atome de zinc, un atome d'aluminium, un atome de magnésium, un atome d'hydrogène, un atome de gallium, un atome de germanium ou un atome d'étain;
et des colorants phthalocyanines de formules générales (III) dans lesquelles
m, R₁, R₂, R₁₀ et M sont définis comme dans la revendication 1 et
Me représente un atome de zinc, un atome d'aluminium, un atome de magnésium, un atome d'hydrogène, un atome de gallium, un atome de germanium ou un atome d'étain;
pour la thérapie photo-dynamique, comme réactifs photo-diagnostiques et pour le traitement de maladies infectieuses causées par des virus, des champignons et des bactéries, du cancer et des maladies dermatologiques.
